# FASCICULE DE BREVET EUROPEEN

(11) **EP 0 521 787 B1**
(45) Date de publication et mention de la délivrance du brevet: **23.04.1997**
(21) Numéro de dépôt: 92401883.1
(22) Date de dépôt: 02.07.1992
(51) Int. Cl.: C07F 9/00, A61K 31/28, A61K 31/555

(54) **Nouveaux complexes de vanadium, leur procédé de préparation et les compositions pharmaceutiques qui les contiennent**
Vanadium Komplexe, ihre Herstellung und pharmazeutische Zusammensetzungen, die sie enthalten
Vanadium complexes, their preparation and pharmaceutical compositions containing them

(30) Priorité: 03.07.1991 FR 9108253
(43) Date de publication de la demande: 07.01.1993
(73) Titulaire: ADIR ET COMPAGNIE, 92415 Courbevoie Cédex (FR)
(72) Inventeur: Lacoste, Jean-Michel, F-92310 Sevres (FR); Duhault, Jacques, F-78290 Croissy sur Seine (FR); Ravel, Denis, F-91430 Igny (FR)

(56) Documents cités:
- EP-A- 0 305 264
- CHEMICAL ABSTRACTS vol. 106, 1987, page 621, abrégé nr. 213521q, Columbus, Ohio, US; K. NAKAJIMA et al.
- CHEMICAL ABSTRACTS vol. 96, no. 26, 28 juin 1982, page 732, abrégé no. 227955w, Columbus, Ohio, US; M. PATEL et al.
- CHEMICAL ABSTRACTS vol. 95, no. 12, 21 septembre 1981, page 679, abrégé no. 107686s, Columbus, Ohio, US; M. PATEL et al.
- CHEMICAL ABSTRACTS vol. 82, no. 4, 27 janvier 1975, page 596, abrégé no. 25282z, Columbus, Ohio, US; A. PASINI et al.
- CHEMICAL ABSTRACTS vol. 80, no. 16, 22 avril 1974, page 526, abrégé no. 90490p, Columbus, Ohio, US; R.L. FARMER et al.
- JOURNAL OF THE CHEMICAL SOCIETY no. 6, 1981, pages 1241-1245; G.A. KOLAWOLE et al.

## Description

La présente invention concerne de nouveaux complexes de vanadium, leur procédé de préparation et les compositions pharmaceutiques qui les contiennent.

Il a été montré que le vanadate de sodium administré par voie orale possédait une efficacité antidiabétique (Science, 227, 1474, 1985). Or l'état diabétique est caractérisé par un défaut de pénétration du glucose dans les cellules dû soit à l'absence d'insuline (diabète insulino-dépendant), soit à une tolérance au glucose diminuée ou à une diminution de l'efficacité de l'insuline au niveau des tissus périphériques (diabète non insulino-dépendant), associé à une élévation de la glycémie. L'administration d'insuline ou de substances insulino-mimétiques peut corriger ces états diabétiques. C'est le cas en particulier du vanadate de sodium ainsi que des complexes de vanadium décrits dans les brevets EP 305264, JP 2-292217. Ces différents complexes activent le transport du glucose et son métabolisme. Cependant le vanadate de sodium présente une intolérance digestive qui rend difficile, dans la plupart des cas, l'absorption des doses nécessaires à l'obtention de concentrations sanguines actives.

D'autre part, d'autres complexes de vanadium ont été décrits dans la littérature sans qu'aucune activité pharmacologique n'ait été mise en évidence. C'est le cas plus particulièrement des composés cités dans les documents suivants : Chem. Lett., 1986 (9), 1483-1486 ; Chem. Abst. vol. 106 (25), 621, n° 213521q ; J. Chem. Soc., 6, 1981, 1241-1245; Chem. Abst., vol. 96 (26), 732, n° 227955w ; Chem. Abst., vol. 95 (12), 679, n°107686s ; Chem. Abst., vol. 82 (4), 596, n° 25282z ; Chem. Abst., vol. 80 (16), 526, n° 90490p.

Les complexes de vanadium décrits dans la présente invention, outre le fait qu'ils soient nouveaux, présentent l'avantage d'être mieux tolérés, moins toxiques et ont démontré une plus grande efficacité que les composés décrits dans l'art antérieur, essentiellement due à une meilleure biodisponibilité de l'entité biologiquement active.

L'invention concerne plus particulièrement de nouveaux complexes de vanadium répondant à la formule (I): 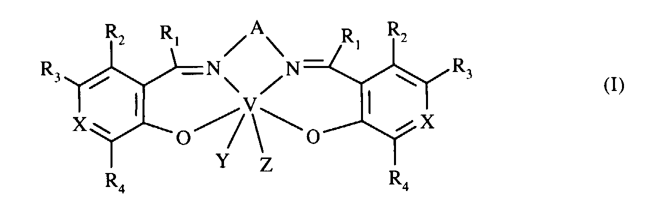 dans laquelle :
- R₁: ou R₄, identiques ou différents, représentent un atome d'hydrogène ou un groupement alkyle (C₁-C₆) linéaire ou ramifié,
- R₂: représente un atome d'hydrogène, un groupement alkyle (C₁-C₆) linéaire ou ramifié, un groupement hydroxyméthyl, un groupement -CH₂OPO(OH)₂ ou un groupement -CH₂OPO(ONa)₂,
- R₃: représente un atome d'hydrogène, un groupement alkyle (C₁-C₆) linéaire ou ramifié, un groupement hydroxy ou l'un quelconque des groupements suivants :

-T-(CH₂)ₙ-CO₂H

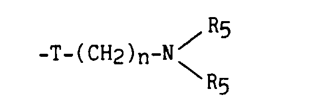

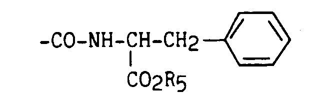

dans lesquels :
T représente un atome d'oxygène ou de soufre,
n représente un entier compris entre 1 et 4,
R₅ représente un atome d'hydrogène ou un groupement alkyle (C₁-C₆) linéaire ou ramifié,
- X: représente un atome d'azote, un radical CH ou un radical CR'₃ (dans lequel R'₃ a la même signification que R₃ à l'exception du cas où R₃ représente un groupement hydroxy),
- A: représente un radical alkylène de formule -(CH₂)ₚ- dans lequel p représente un entier compris entre 2 et 4, éventuellement substitué par un ou plusieurs groupements alkyles (C₁-C₄) linéaires ou ramifiés, ou l'un quelconque des radicaux suivants : 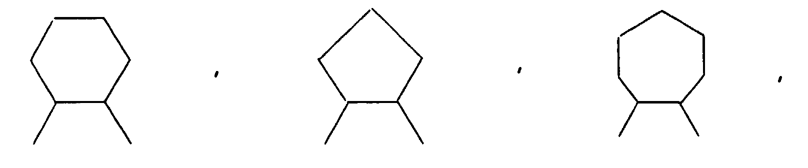
- Y et Z: forment ensemble un atome d'oxygène et dans ce cas le vanadium dans le complexe de formule (I) se trouve au degré d'oxydation IV
à la condition que :
lorsque :
X représente un radical CH ou C-alkyle,
alors R₃ est différent d'un atome d'hydrogène ou d'un groupement alkyle (C₁-C₆) linéaire ou ramifié,
leurs isomères, énantiomères, diastéréoisomères et épimères ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

L'invention s'étend aussi au procédé de préparation des composés de formule (I) caractérisé en ce que l'on fait réagir deux équivalents d'un composé de formule (II) : 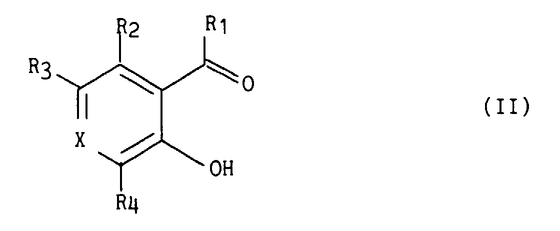 dans laquelle R₁, R₂, R₃ et R₄ ont la même signification que dans la formule (I),
avec un équivalent d'un composé de formule (III), sous atmosphère inerte : 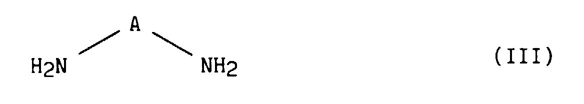 dans laquelle A a la même signification que dans la formule (I),
pour conduire au composé de formule (IV) dont on sépare éventuellement les isomères par une technique classique de séparation, que l'on purifie le cas échéant par une technique classique de purification : 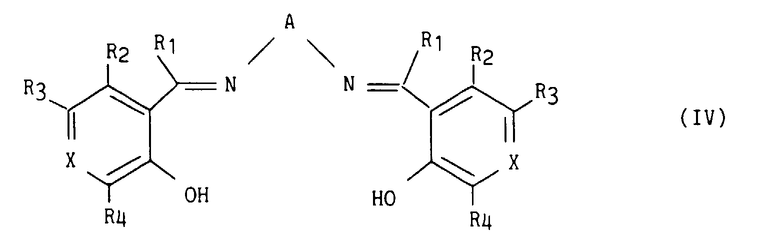 dans laquelle R₁, R₂, R₃, R₄, A et X ont la même signification que dans la formule (I),
que l'on traite :
- soit par une solution aqueuse pentahydratée de sulfate de vanadyle en milieu dichlorométhane, ou bien après traitement par de la soude par une solution aqueuse pentahydratée de sulfate de vanadyle en milieu tétrahydrofurane, ou bien par du sulfate de vanadyle en milieu diméthylformamide, pour conduire au complexe de formule (I/a), cas particulier des composés de formule (I) dans lequel le vanadium se trouve au degré d'oxydation IV : 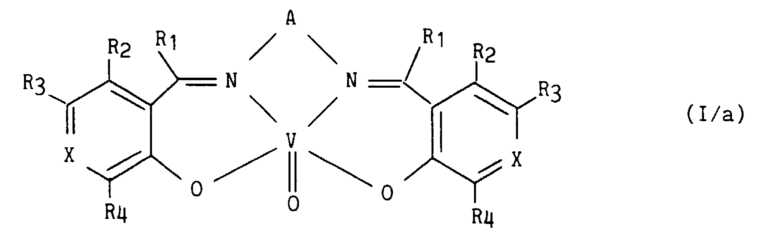
- soit par une solution de sulfate de vanadyle dans le diméthylformamide en présence d'oxygène selon la technique décrite par H.J.BIELIG et Coll. (Liebigs Ann.Chem., 580, 135, 1953)
pour conduire au complexe de formule (I/b), cas particulier des composés de formule (I), dans lequel le vanadium se trouve au degré d'oxydation V : 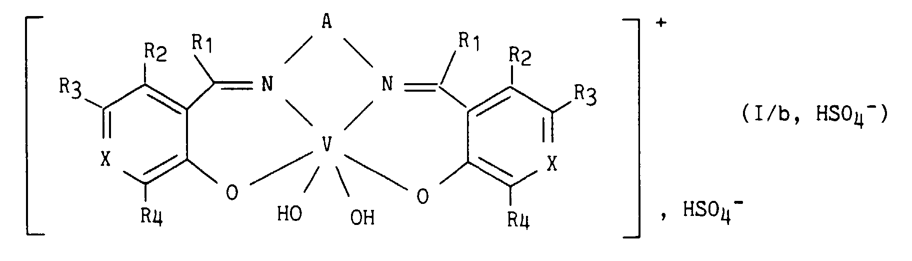 composés de formule (I/a) ou (I/b) que l'on purifie, le cas échéant, par une technique classique de purification et que l'on transforme, si on le désire, en leurs sels d'addition à un acide ou à une base pharmaceutiquement acceptable.

Les composés de l'invention possèdent des propriétés pharmacologiques très intéressantes. Ils possèdent des activités insulino-mimétiques non seulement in vitro mais également in vivo. Les résultats obtenus aussi bien lors de la mesure du métabolisme du glucose et la capture de 2-désoxyglucose que lors d'essais effectués sur des souris génétiquement insulino-résistantes ou sur des rats rendus diabétiques par injection de streptozotocine, montrent que les composés de l'invention sont utilisables dans le traitement des états d'insulino-résistance associés ou non à une hyperglycémie et une hyperinsulinémie tels que diabètes de type I et II, obésité et hypertension.

Une conséquence favorable du traitement par ces composés est la réduction des lipides sanguins pouvant concourir à la prévention des macroangiopathies.

La présente invention s'étend également à l'utilisation du N,N'-di(salicylidène)éthylène diamine, oxovanadium (IV) (décrit par P. Pfeiffer et coll., J. für Praktische Chemie, 149, 217, 1937) pour l'obtention de compositions pharmaceutiques utiles dans le traitement du diabète.

La présente invention a également pour objet les compositions pharmaceutiques renfermant comme principe actif au moins un composé de formule générale (I) ou un de ses sels d'addition à un acide pharmaceutiquement acceptable, seul ou en combinaison avec un ou plusieurs excipients ou véhicules inertes, non toxiques.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les sachets, les paquets, les gélules, les suppositoires, crèmes, pommades, gels dermiques, les aérosols.

La posologie varie selon l'âge et le poids du patient, la nature et la sévérité de l'affection ainsi que la voie d'administration. Celle-ci peut être orale, nasale, rectale ou parentérale. D'une manière générale, elle s'échelonne entre 100 mg et 1 g pour un traitement en une ou plusieurs prises par 24 heures.

Les exemples suivants illustrent l'invention et ne la limitent en aucune façon.

Les préparations A à E ne permettent pas d'obtenir les composés de l'invention mais conduisent à des intermédiaires utiles lors de la synthèse des composés de l'invention.

### Préparation A : 2-Hydroxy-4-[2-(diméthylamino)éthoxy]benzaldéhyde

Un mélange contenant 0,8 mole de 2,4-dihydroxybenzaldéhyde, 0,8 mole de 2-chloro-1-diméthylamino éthane et 1,6 mole de carbonate de potassium dans 1200 ml de méthyléthylcétone anhydre est porté à reflux 1 heure 30 sous agitation.

Après refroidissement, le précipité formé est filtré, lavé par de la méthyléthylcétone. Le filtrat est ensuite évaporé et conduit à une huile brune qui est purifiée par chromatographie sur colonne de silice en utilisant comme solvant d'élution un mélange dichlorométhane/méthanol (90/10). Le produit attendu est alors obtenu après recristallisation dans de l'isopropanol.
Point de fusion : 82-83°C

| Microanalyse élémentaire : | | | |
|---|---|---|---|
| | C % | H % | N % |
| calculé | 63,14 | 7,23 | 6,69 |
| trouvé | 63,24 | 7,15 | 6,58 |

### Préparation B : 2-Hydroxy-5-[2-(diméthylamino)éthoxy]benzaldéhyde

Le produit attendu est obtenu sous forme d'une huile jaune pâle en utilisant le même procédé que celui décrit dans la préparation A mais en remplacant le 2,4-dihydroxybenzaldéhyde par le 2,5-dihydroxybenzaldéhyde.

| Microanalyse élémentaire : | | | |
|---|---|---|---|
| | C % | H % | N % |
| calculé | 63,14 | 7,23 | 6,69 |
| trouvé | 62,92 | 7,17 | 6,73 |

### Préparation C : 2-Hydroxy-4-(éthoxycarbonylméthoxy)benzaldéhyde

En procédant comme dans la préparation A mais en remplacant le 2-chloro-1-diméthylaminoéthane par le bromoacétate d'éthyle et en portant l'ensemble à reflux pendant 3 heures, on obtient le produit attendu qui est purifié par cristallisation du résidu huileux dans du toluène et conduit à une poudre blanche.
Point de fusion : 97-99°C

| Microanalyse élémentaire : | | |
|---|---|---|
| | C % | H % |
| calculé | 58,93 | 5,39 |
| trouvé | 59,19 | 5,50 |

### Préparation D : N-(3-Formyl-4-hydroxybenzoyl)phénylalanine méthyl ester

A une suspension, sous agitation, contenant 80 mmoles d'acide 3-formyl-4-hydroxybenzoïque (préparé selon H.WYMBERG, J. Am.Chem.Soc., 76, 4998, 1954), 87 mmoles de N-hydroxysuccinimide dans 320 ml de chloroforme, refroidie à 10°C, sont ajoutées 87 mmoles de dicyclohexylcarbodiimide. Après retour à température ambiante, l'ensemble est maintenu une nuit sous agitation. Le précipité est alors filtré. Le filtrat est refroidi à 8°C et traité, goutte à goutte, sous agitation par une suspension contenant 160 mmoles de chlorhydrate de phénylalanine méthyl ester et 160 mmoles de triéthylamine dans 100 ml de diméthylformamide anhydre. Le mélange est agité 3 heures à 20°C puis 5 heures à 50°C. Après refroidissement et évaporation des solvants, le résidu est repris par 400 ml d'acétate d'éthyle. Après lavage de cette solution par de l'acide chlorhydrique 1N puis par de l'eau, la phase organique est séchée et évaporée. Le résidu huileux conduit au produit attendu par cristallisation dans du toluène.
Point de fusion : 128-129°C

| Microanalyse élémentaire : | | | |
|---|---|---|---|
| | C % | H % | N % |
| calculé | 66,05 | 5,23 | 4,28 |
| trouvé | 66,21 | 5,34 | 4,76 |

### Préparation E : N-(4-Formyl-3-hydroxybenzoyl)phénylalanine méthyl ester

Le produit attendu est obtenu en procédant comme dans la préparation D mais en remplacant l'acide 3-formyl-4-hydroxybenzoïque par l'acide 4-formyl-3-hydroxybenzoïque (préparé selon T.L.HULLAR et coll. J.Med.Chem., 12, 420, 1968).
Point de fusion : 131-133°C

| Microanalyse élémentaire : | | | |
|---|---|---|---|
| | C % | H % | N % |
| calculé | 66,05 | 5,23 | 4,28 |
| trouvé | 66,21 | 5,34 | 4,76 |

### EXEMPLE 1 :

**N,N'-di-[4-(2-diméthylaminoéthoxy) salicylidène] éthylène diamine, oxovanadium (IV)**

### STADE A : N, N'-di-[4-(2-diméthylaminoéthoxy)salicylidène]éthylène diamine

A une solution contenant 50 mmoles du composé obtenu dans la préparation A dans 50 ml d'éthanol anhydre sont ajoutées goutte à goutte, sous atmosphère d'azote 25 mmoles d'éthylène diamine. L'ensemble est porté une heure à reflux.

Après refroidissement et évaporation du solvant, le produit attendu est obtenu après recristallisation du résidu dans du cyclohexane.
Rendement : 84 %
Point de fusion : 92-94°C

| Microanalyse élémentaire : | | | |
|---|---|---|---|
| | C % | H % | N % |
| calculé | 65,14 | 7,74 | 12,66 |
| trouvé | 65,00 | 7,72 | 12,62 |

### STADE B : N,N'-di-[4-(2-diméthylaminoéthoxy)salicylidène]éthylène diamine, oxovanadium (IV)

A une solution, contenant 14 mmoles du composé obtenu au stade A dans 50 ml de dichlorométhane est ajoutée, sous agitation, à température ambiante, une solution contenant 14 mmoles de sulfate de vanadyle pentahydraté dans 40 ml d'eau distillée. L'ensemble est agité 30 minutes puis décanté. La phase aqueuse de couleur verte est diluée par 50 ml d'eau distillée, puis filtrée. Le filtrat est traité par de la triéthylamine jusqu'à un pH basique accompagné d'une précipitation. La phase aqueuse et le précipité sont extraits par du dichlorométhane. Cette phase organique est alors lavée par de l'eau, séchée, puis évaporée.

Le produit attendu est obtenu par recristallisation du résidu solide dans le toluène.
Rendement : 84 %
Point de fusion : 190-192°C

| Microanalyse élémentaire : | | | | |
|---|---|---|---|---|
| | C % | H % | N % | V % |
| calculé | 56,80 | 6,36 | 11,04 | 10,04 |
| trouvé | 56,60 | 6,33 | 10,97 | 10,26 |

Les exemples suivants ont été obtenus en utilisant le même mode opératoire que celui décrit dans l'exemple 1.

### EXEMPLE 2 :

**N,N'-di-[4-(2-diméthylaminoéthoxy)salicylidène]propylène diamine, oxovanadium (IV)**

### STADE A : N, N'-di-[4-(2-diméthylaminoéthoxy)salicylidène]propylène diamine

Ce stade est identique au stade A de l'exemple 1 mais l'éthylène diamine est remplacée par la propylène diamine.
Rendement : 82 %
Point de fusion : 63-65°C

| Microanalyse élémentaire : | | | |
|---|---|---|---|
| | C % | H % | N % |
| calculé | 65,77 | 7,95 | 12,27 |
| trouvé | 65,24 | 8,08 | 12,57 |

### STADE B : N,N'-di-[4-(2-diméthylaminoéthoxy)salicylidène]propylène diamine, oxovanadium (IV)

Ce stade B est identique au stade B de l'exemple 1.
Rendement : 82 %
Point de fusion : 208-211°C

| Microanalyse élémentaire : | | | |
|---|---|---|---|
| | C % | H % | N % |
| calculé | 57,58 | 6,57 | 10,74 |
| trouvé | 57,31 | 6,45 | 10,81 |

### EXEMPLE 3 :

### N,N'-di-[4-(2-diméthylaminoéthoxy)salicylidène](trans-1,2-cyclohexane)diamine, oxovanadium (IV)

### STADE A : N,N'-di-[4-(2-diméthylaminoéthoxy)salicylidène] (trans-1,2-cyclohexane)diamine

Le produit attendu est obtenu, sous forme d'huile, en procédant comme au stade A de l'exemple 1 mais en remplacant l'éthylène diamine par le (trans-1,2-cyclohexane)diamine.
Rendement : 77 %

| Microanalyse élémentaire : | | | |
|---|---|---|---|
| | C % | H % | N % |
| calculé | 67,44 | 8,49 | 11,24 |
| trouvé | 67,33 | 8,57 | 11,05 |

### STADE B : N,N'-di-[4-(2-diméthylaminoéthoxy)salicylidène] (trans-1,2-cyclohexane)diamine, oxovanadium (IV)

Le stade B est identique au stade B de l'exemple 1.
Rendement : 80 %
Point de fusion : 178-182°C

| Microanalyse élémentaire : | | | |
|---|---|---|---|
| | C % | H % | N % |
| calculé | 59,89 | 6,82 | 9,98 |
| trouvé | 59,89 | 6,72 | 9,76 |

### EXEMPLE 4 :

### N,N'-di-[5-(2-diméthylaminoéthoxy)salicylidène]éthylène diamine, oxovanadium (IV)

### STADE A : N,N'-di-[5-(2-diméthylaminoéthoxy)salicylidène] éthylène diamine

Le produit attendu est obtenu en procédant comme au stade A de l'exemple 1 mais en utilisant comme produit de départ le composé obtenu dans la préparation B à la place du composé obtenu dans la préparation A.
Rendement : 72 %
Point de fusion : 84-86°C

| Microanalyse élémentaire : | | | |
|---|---|---|---|
| | C % | H % | N % |
| calculé | 65,14 | 7,74 | 12,66 |
| trouvé | 64,93 | 8,15 | 12,78 |

### STADE B : N,N'-di-[5-(2-diméthylaminoéthoxy)salicylidène] éthylène diamine, oxovanadium (IV)

Le stade B est identique au stade B de l'exemple 1.
Rendement : 75 %
Point de fusion : 180-184°C

| Microanalyse élémentaire : | | | |
|---|---|---|---|
| | C % | H % | N % |
| calculé | 56,80 | 6,36 | 11,04 |
| trouvé | 56,75 | 6,22 | 11,02 |

### EXEMPLE 5:

### N,N'-di(3,4,6-triméthyl-5-hydroxy salicylidène) éthylène diamine, oxovanadium (IV)

### STADE A : N, N'-di(3,4,6-triméthyl-5-hydroxy salicylidène) éthylène diamine

Le produit attendu est obtenu en procédant comme au stade A de l'exemple 1 mais en utilisant comme produit de départ le 3,4,6-triméthyl-2,5-hydroxy benzaldéhyde obtenu comme décrit par A.MAYER et coll. (Helvetica Chem.Acta, XLVI (II), 67, 650, 1963) à la place du composé décrit dans la préparation A.
Rendement : 86 **%**
Point de fusion : 225-229°C

| Microanalyse élémentaire : | | | |
|---|---|---|---|
| | C % | H % | N % |
| calculé | 68,73 | 7,34 | 7,29 |
| trouvé | 68,23 | 7,41 | 7,02 |

### STADE B : N,N'-di(3,4,6-triméthyl-5-hydroxy salicylidène) éthylène diamine, oxovanadium (IV)

Le stade B est identique au stade B de l'exemple 1.
Rendement : 81 **%**
Point de fusion : > 250° C

| Microanalyse élémentaire : | | | |
|---|---|---|---|
| | C % | H % | N % |
| calculé | 58,80 | 5,83 | 6,23 |
| trouvé | 58,64 | 5,48 | 6,59 |

### EXEMPLE 6 :

### N,N'-di[5-[(phénylalanine méthyl ester) carbonyl] salicylidène] éthylène diamine, oxovanadium (IV)

### STADE A : N,N'-di[5-[(phénylalanine méthyl ester) carbonyl] salicylidène]éthylène diamine

Le produit attendu est obtenu en procédant comme au stade A de l'exemple 1 mais en utilisant comme produit de départ, le composé obtenu dans la préparation D à la place du composé obtenu dans la préparation A.
Rendement : 70 %
Point de fusion : 186-187°C

| Microanalyse élémentaire : | | | |
|---|---|---|---|
| | C % | H % | N % |
| calculé | 67,24 | 5,64 | 8,25 |
| trouvé | 66,99 | 5,69 | 8,40 |

### STADE B : N,N'-di[5-[(phénylalanine méthyl ester) carbonyl] salicylidène]éthylène diamine, oxovanadium (IV)

Le stade B est identique au stade B de l'exemple 1.
Rendement : 64 %
Point de fusion : 154-162°C

| Microanalyse élémentaire : | | | |
|---|---|---|---|
| | C % | H % | N % |
| calculé | 61,37 | 4,88 | 7,53 |
| trouvé | 61,25 | 4,80 | 7,56 |

### EXEMPLE 7:

### N,N'-di[4-[(phénylalanine méthyl ester) carbonyl) salicylidène] éthylène diamine, oxovanadium (IV)

### STADE A : N,N'-di[4-[(phénylalanine méthyl ester) carbonyl) salicylidène]éthylène diamine

Le produit attendu est obtenu en procédant comme au stade A de l'exemple 1 mais en utilisant comme produit de départ, le composé obtenu dans la préparation E à la place du composé obtenu dans la préparation A.
Rendement : 72 %
Point de fusion : 162-166°C

| Microanalyse élémentaire : | | | |
|---|---|---|---|
| | C % | H % | N % |
| calculé | 67,24 | 5,64 | 8,25 |
| trouvé | 67,05 | 5,51 | 8,22 |

### STADE B : N,N'-di[4-[(phénylalanine méthyl ester) carbonyl) salicylidène]éthylène diamine, oxovanadium (IV)

Le stade B est identique au stade B de l'exemple 1.
Rendement : 56 %
Point de fusion :252-256°C

| Microanalyse élémentaire : | | | |
|---|---|---|---|
| | C % | H % | N % |
| calculé | 61,37 | 4,88 | 7,53 |
| trouvé | 60,52 | 4,81 | 7,59 |

### EXEMPLE 8 :

### [N,N'-(bis-pyridoxal)éthylène diimine]oxovanadium (IV)

### STADE A : N,N'-(bis-pyridoxal)éthylène diimine

Le produit attendu est obtenu comme décrit dans le brevet EP-292761.

### STADE B : [N,N'-(bis-pyridoxal)éthylène diimine]oxovanadium (IV)

Le stade B est identique au stade B de l'exemple 1.
Rendement : 82 %
Point de fusion : > 250°C

| Microanalyse élémentaire : | | | |
|---|---|---|---|
| | C % | H % | N % |
| calculé | 51,07 | 4,76 | 13,23 |
| trouvé | 50,77 | 4,87 | 13,23 |

### EXEMPLE 9:

### N,N'-di-[4-(carboxyméthyloxy) salicylidène] éthylène diamine, oxovanadium (IV), sel de diterbutylamine

### STADE A : N,N'-di-[4-(éthoxycarbonyl méthyloxy) salicylidène] diamine

Le produit attendu est obtenu en procédant comme au stade A de l'exemple 1 mais en utilisant comme produit de départ le composé obtenu dans la préparation C à la place du composé obtenu dans la préparation A.
Rendement : 80 %
Point de fusion : 104-106°C

| Microanalyse élémentaire : | | | |
|---|---|---|---|
| | C % | H % | N % |
| calculé | 61,01 | 5,97 | 5,93 |
| trouvé | 60,87 | 6,05 | 5,85 |

### STADE B : N,N'-di-[4-(carboxyméthyloxy) salicylidène] éthylène diamine, oxovanadium (IV), sel de diterbutylamine

A une suspension contenant 16 mmoles du produit obtenu au stade A dans 100 ml d'un mélange tétrahydrofurane/eau (50/50) sont additionnés 20 ml de NaOH 1N à température ambiante. Après 4 heures d'agitation le tétrahydrofurane est évaporé ; la phase aqueuse résiduelle est lavée par du dichlorométhane et traitée par une solution contenant 10 mmoles de sulfate de vanadyle pentahydraté dans 20 ml d'eau distillée. Le mélange réactionnel est maintenu sous agitation, à température ambiante, pendant une heure 30 minutes puis acidifié par de l'acide chlorhydrique 3N jusqu'à pH 3-4. Le précipité formé est filtré, lavé par de l'eau jusqu'à neutralité et séché.

Le sel de tertbutylamine correspondant est formé par agitation du précipité formé dans une solution aqueuse de tertbutylamine et purifié par recristallisation dans un mélange eau/acétone (30/70).
Rendement : 53 %
Point de fusion : 235-240°C

| Microanalyse élémentaire : | | | |
|---|---|---|---|
| | C % | H % | N % |
| calculé | 53,59 | 6,42 | 8,93 |
| trouvé | 53,55 | 6,17 | 8,28 |

### EXEMPLE 10 :N,N'-bis[(5-pyridoxal phosphate) éthylène diimine] oxovanadium (IV), sel tétrasodique

### STADE A : N,N'-(bis-pyridoxal phosphate)éthylène diimine, sel tétrasodique

Le produit attendu est obtenu comme décrit dans le brevet EP 290047.

### STADE B : N,N'-bis[(5-pyridoxal phosphate) éthylène diimine] oxovanadium (IV), sel tétrasodique

Une solution contenant 10 mmoles du produit obtenu au stade A dans 20 ml d'eau distillée est traitée à température ambiante, sous agitation, par une solution contenant 10 mmoles de sulfate de vanadyle pentahydraté dans 15 ml d'eau distillée.

Après une heure d'agitation, le précipité formé est filtré, séché puis repris par 100 ml d'eau. La suspension est traitée par 12 ml de NaOH 1N. La solution est filtrée et évaporée. Le produit attendu est obtenu par recristallisation du résidu dans un mélange eau/éthanol (30/70).
Rendement : 76 %
Point de fusion : > 250°C

| Microanalyse élémentaire : | | | |
|---|---|---|---|
| | C % | H % | N % |
| calculé | 32,21 | 2,70 | 8,35 |
| trouvé | 32,55 | 3,79 | 7,85 |

### ETUDE PHARMACOLOGIQUE DES COMPOSES DE L'INVENTION

### EXEMPLE 11:

### Effets insulino-mimétiques in vitro

Les effets insulino-mimétiques des composés de l'invention ont été étudiés, in vitro, sur des fragments de tissu adipeux en mesurant le métabolisme du glucose marqué au carbone 14 et la capture de 2-désoxyglucose selon les techniques décrites respectivement par M.RODBELL (J. Biol.Chem., 239, 375, 1964) et par J.M.OLEFSKY (J.Clin.Invest., 56, 1499, 1975).

Les résultats obtenus avec les composés de l'invention ont été comparés à la réponse à l'insuline qui représente + 100 % à 10⁻⁹M. Le produit de référence utilisé est l'orthovanadate de sodium.

Les résultats obtenus à 10⁻⁴M sont regroupés dans le tableau ci-dessous :

| Ex | Métabolisme du glucose marqué | Capture du 2-désoxyglucose |
|---|---|---|
| Ex 1 | + 43 % | + 78 % |
| Ex 3 | + 51 % | + 96 % |
| Ex 4 | + 56 % | + 113 % |
| Orthovanadate de sodium | + 23 % | + 95 % |

Ces résultats montrent que le métabolisme du glucose marqué et la capture du 2-désoxyglucose par le tissu adipeux sont augmentés par la présence d'insuline (+ 100 %) ou des composés cités.

### EXEMPLE 12 :

### Réponse hypoglycémique in vivo

La réponse hypoglycémique a été étudiée après administration par voie orale des composés de l'invention, après mise en suspension dans une solution à 20 % de gomme du Sénégal, à des rats rendus diabétiques par injection de streptozotocine (65 mg/kg) selon la technique décrite par A.JUNOD et coll. (J.Clin.Invest., 48, 11, 2129, 1969).

Les résultats indiquant la baisse de la glycémie, observée après 10 jours de traitement par les composés de l'invention à la dose de 2 x 12,5 mg/kg/jour, sont regroupés dans le tableau ci-dessous.

Le produit de référence utilisé est l'orthovanadate de sodium.

| Composé | Baisse de la glycémie (%) | Quantité de vanadium correspondante (mg/kg) |
|---|---|---|
| Ex 1 | - 45 | 2,50 |
| Ex 5 | - 31 | 2,83 |
| Ex 7 | - 41 | 1,71 |
| Orthovanadate de sodium | - 48 | 6,93 |

Ces résultats montrent que la baisse de la glycémie observée avec les composés cités ci-dessus est comprise entre - 31 % et - 45 % pour des quantités de vanadium 2,5 à 4 fois inférieures à la quantité de vanadium

correspondant à l'administration, dans les mêmes conditions, d'orthovanadate de sodium qui provoque une baisse de la glycémie de - 48 %.

### COMPOSITION PHARMACEUTIQUE

### EXEMPLE 13 :

### Comprimé : formule de préparation pour 1000 comprimés dosés à 100 mg

| | |
|---|---|
| Composé de l'exemple 1 | 100 g |
| Hydroxypropylcellulose | 2 g |
| Amidon de blé | 10 g |
| Lactose | 100 g |
| Stéarate de magnésium | 3 g |
| Talc | 3 g |

## Revendications (Revendications pour l'(les) Etat(s) contractant(s) suivant(s): AT, BE, CH, DE, DK, FR, GB, IT, LI, LU, NL, PT, SE)

1. Composés de formule (I) : 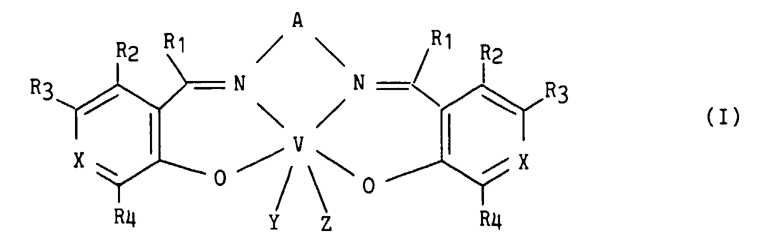 dans laquelle :
R₁ ou R₄, identiques ou différents, représentent un atome d'hydrogène ou un groupement alkyle (C₁-C₆) linéaire ou ramifié,
R₂ représente un atome d'hydrogène, un groupement alkyle (C₁-C₆) linéaire ou ramifié, un groupement hydroxyméthyl, un groupement -CH₂OPO(OH)₂ ou un groupement -CH₂OPO(ONa)₂,
R₃ représente un atome d'hydrogène, un groupement alkyle (C₁-C₆) linéaire ou ramifié, un groupement hydroxy ou l'un quelconque des groupements suivants :
-T-(CH₂)ₙ-CO₂H 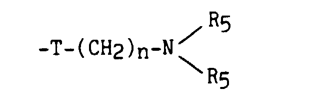 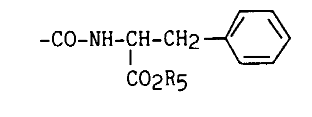
dans lesquels :
T représente un atome d'oxygène ou de soufre,
n représente un entier compris entre 1 et 4,
R₅ représente un atome d'hydrogène ou un groupement alkyle (C₁-C₆) linéaire ou ramifié,
X représente un atome d'azote, un radical CH ou un radical CR'₃ (dans lequel R'₃ a la même signification que R₃ à l'exception du cas où R₃ représente un groupement hydroxy),
A représente un radical alkylène de formule -(CH₂)ₚ- dans lequel p représente un entier compris entre 2 et 4, éventuellement substitué par un ou plusieurs groupements alkyles (C₁-C₄) linéaires ou ramifiés, ou l'un quelconque des radicaux suivants : 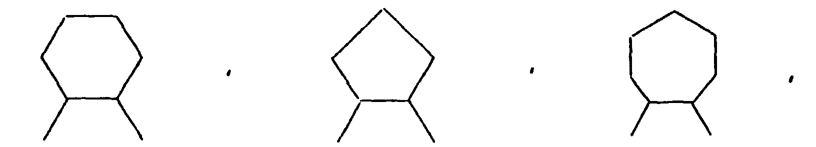
Y et Z forment ensemble un atome d'oxygène et dans ce cas le vanadium dans le complexe de formule (I) se trouve au degré d'oxydation IV
à la condition que :
lorsque :
X représente un radical CH ou C-alkyle,
alors R₃ est différent d'un atome d'hydrogène ou d'un groupement alkyle (C₁-C₆) linéaire ou ramifié,
leurs isomères, énantiomères, diastéréoisomères et épimères ainsi que leurs sels d'addition à un acide ou à une base pharmaceutiquement acceptable.

2. Composés de formule (I) selon la revendication 1 tels que X représente un radical (CH₃)₂N-CH₂-CH₂-O-C, leurs isomères, énantiomères, diastéréoisomères et épimères ainsi que leurs sels d'addition à un acide ou à une base pharmaceutiquement acceptable.

3. Composés de formule (I) selon la revendication 1 tels que X représente un atome d'azote, leurs énantiomères, diastéréoisomères et épimères ainsi que leurs sels d'addition à un acide ou à une base pharmaceutiquement acceptable.

4. Composés de formule (I) selon la revendication 1 tels que A représente un radical éthylène, leurs énantiomères, diastéréoisomères et épimères ainsi que leurs sels d'addition à un acide ou à une base pharmaceutiquement acceptable.

5. Composés de formule (I) selon la revendication 1 tels que Y et Z forment ensemble un atome d'oxygène, leurs énantiomères, diastéréoisomères et épimères ainsi que leurs sels d'addition à un acide ou à une base pharmaceutiquement acceptable.

6. Composé de formule (I) selon l'une quelconque des revendications 1, 2, 4 et 5 qui est le N,N'-di-[4-(2-diméthylaminoéthoxy)salicylidène]éthylène diamine, oxovanadium (IV), ainsi que ses sels d'addition à un acide pharmaceutiquement acceptable.

7. Procédé de préparation des composés de formule (I) selon la revendication 1 caractérisé en ce que l'on fait réagir deux équivalents d'un composé de formule (II) : 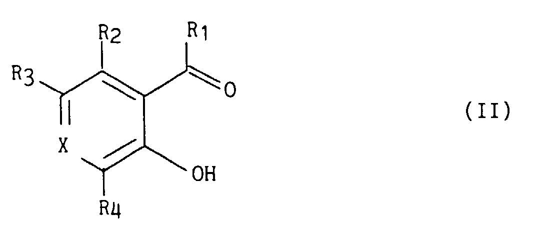 dans laquelle R₁, R₂, R₃ et R₄ ont la même signification que dans la formule (I),
avec un équivalent d'un composé de formule (III), sous atmosphère inerte : 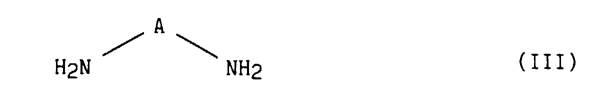 dans laquelle A a la même signification que dans la formule (I),
pour conduire au composé de formule (IV) dont on sépare éventuellement les isomères par une technique classique de séparation, que l'on purifie le cas échéant par une technique classique de purification : 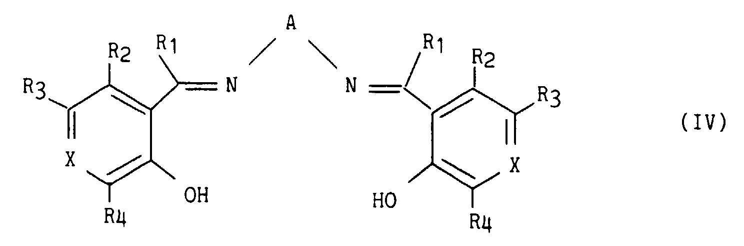 dans laquelle R₁, R₂, R₃, R₄, A et X ont la même signification que dans la formule (I),
que l'on traite :
- soit par une solution aqueuse pentahydratée de sulfate de vanadyle en milieu dichlorométhane, ou bien après traitement par de la soude par une solution aqueuse pentahydratée de sulfate de vanadyle en milieu tétrahydrofurane, ou bien par du sulfate de vanadyle en milieu diméthylformamide,
pour conduire au complexe de formule (I/a), cas particulier des composés de formule (I), dans lequel le vanadium se trouve au degré d'oxydation IV : 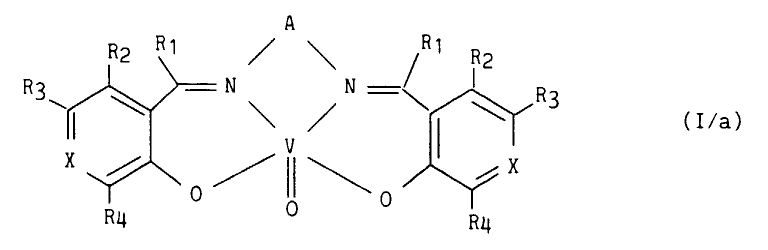
- soit par une solution alcoolique de sulfate de vanadyle en présence d'oxygène,
pour conduire au composé de formule (I/b), cas particulier des composés de formule (I), dans lequel le vanadium se trouve au degré d'oxydation V : 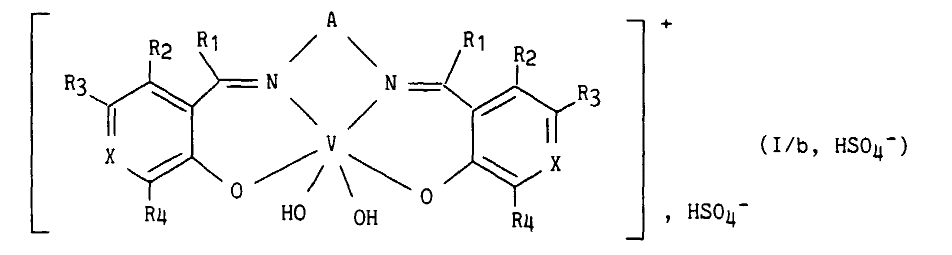 composés de formule (I/a) ou (I/b) que l'on purifie, le cas échéant, par une technique classique de purification et que l'on transforme, si on le désire, en leurs sels d'addition à un acide ou à une base pharmaceutiquement acceptable.

8. Compositions pharmaceutiques contenant comme principe actif au moins un composé selon l'une quelconque des revendications 1 à 6, seul ou en combinaison avec un ou plusieurs excipients ou véhicules inertes, non toxiques, pharmaceutiquement acceptables.

9. Compositions pharmaceutiques selon la revendication 8 contenant au moins un principe actif selon l'une quelconque des revendications 1 à 6 utiles dans le traitement du diabète, de l'obésité et des pathologies associées.

10. Utilisation de la N,N'-di(salicylidène) éthylène diamine, oxovanadium (IV), pour l'obtention d'une composition pharmaceutique selon la revendication 8 utilisable dans le traitement du diabète, de l'obésité et des pathologies associées.

## Revendications (Revendications pour l'(les) Etat(s) contractant(s) suivant(s): GR)

1. Procédé de préparation des composés de formule (I) : 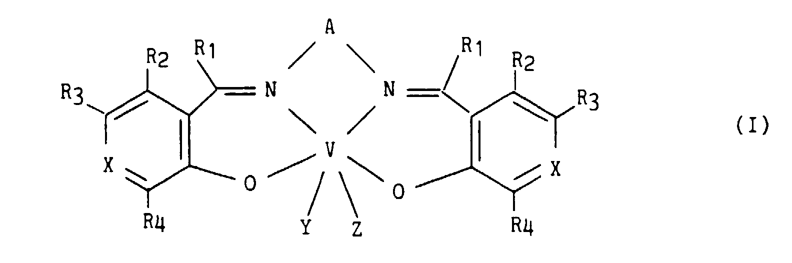 dans laquelle :
R₁ ou R₄, identiques ou différents, représentent un atome d'hydrogène ou un groupement alkyle (C₁-C₆) linéaire ou ramifié,
R₂ représente un atome d'hydrogène, un groupement alkyle (C₁-C₆) linéaire ou ramifié, un groupement hydroxyméthyl, un groupement -CH₂OPO(OH)₂ ou un groupement -CH₂OPO(ONa)₂,
R₃ représente un atome d'hydrogène, un groupement alkyle (C₁-C₆) linéaire ou ramifié, un groupement hydroxy ou l'un quelconque des groupements suivants :
-T-(CH₂)ₙ-CO₂H 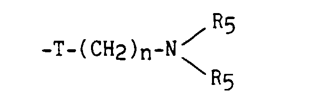 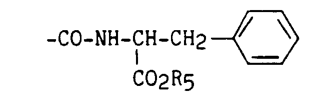
dans lesquels :
T représente un atome d'oxygène ou de soufre,
n représente un entier compris entre 1 et 4,
R₅ représente un atome d'hydrogène ou un groupement alkyle (C₁-C₆) linéaire ou ramifié,
X représente un atome d'azote, un radical CH ou un radical CR'₃ (dans lequel R'₃ a la même signification que R₃ à l'exception du cas où R₃ représente un groupement hydroxy),
A représente un radical alkylène de formule -(CH₂)ₚ- dans lequel p représente un entier compris entre 2 et 4, éventuellement substitué par un ou plusieurs groupements alkyles (C₁-C₄) linéaires ou ramifiés, ou l'un quelconque des radicaux suivants : 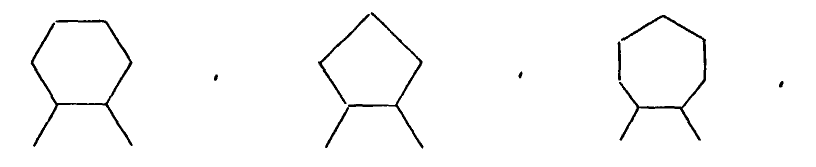
Y et Z forment ensemble un atome d'oxygène et dans ce cas le vanadium dans le complexe de formule (I) se trouve au degré d'oxydation IV
à la condition que :
lorsque :
X représente un radical CH ou C-alkyle,
alors R₃ est différent d'un atome d'hydrogène ou d'un groupement alkyle (C₁-C₆) linéaire ou ramifié,
leurs isomères, énantiomères, diastéréoisomères et épimères ainsi que leurs sels d'addition à un acide ou à une base pharmaceutiquement acceptable,
caractérisé en ce que l'on fait réagir deux équivalents d'un composé de formule (II) : 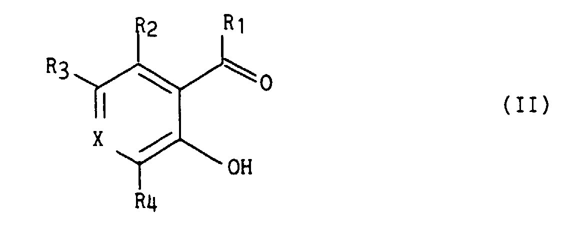 dans laquelle R₁, R₂, R₃ et R₄ ont la même signification que dans la formule (I),
avec un équivalent d'un composé de formule (III), sous atmosphère inerte : 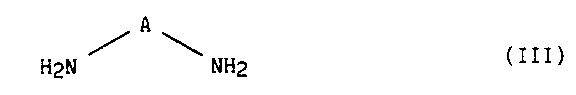 dans laquelle A a la même signification que dans la formule (I),
pour conduire au composé de formule (IV) dont on sépare éventuellement les isomères par une technique classique de séparation, que l'on purifie le cas échéant par une technique classique de purification : 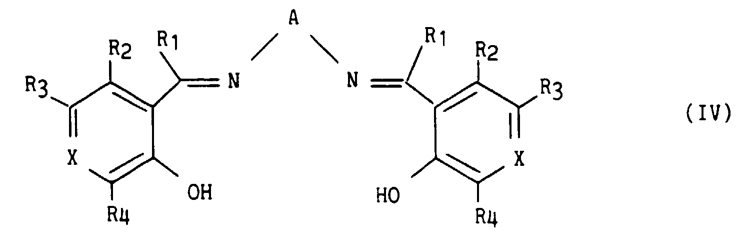 dans laquelle R₁, R₂, R₃, R₄, A et X ont la même signification que dans la formule (I),
que l'on traite :
- soit par une solution aqueuse pentahydratée de sulfate de vanadyle en milieu dichlorométhane, ou bien après traitement par de la soude par une solution aqueuse pentahydratée de sulfate de vanadyle en milieu tétrahydrofurane, ou bien par du sulfate de vanadyle en milieu diméthylformamide,
pour conduire au complexe de formule (I/a), cas particulier des composés de formule (I), dans lequel le vanadium se trouve au degré d'oxydation IV : 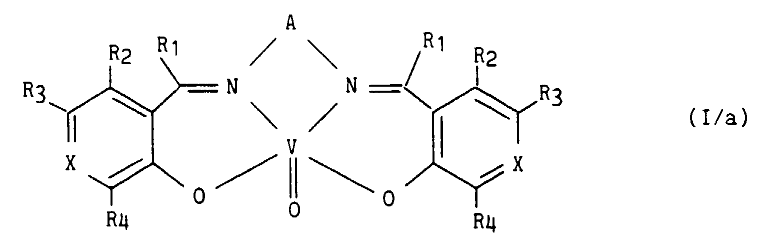
- soit par une solution alcoolique de sulfate de vanadyle en présence d'oxygène,
pour conduire au composé de formule (I/b), cas particulier des composés de formule (I), dans lequel le vanadium se trouve au degré d'oxydation V : 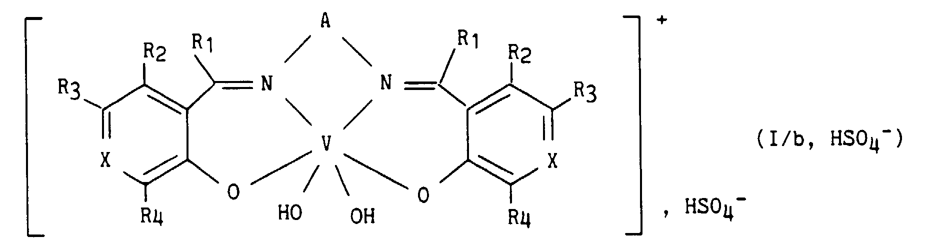 composés de formule (I/a) ou (I/b) que l'on purifie, le cas échéant, par une technique classique de purification et que l'on transforme, si on le désire, en leurs sels d'addition à un acide ou à une base pharmaceutiquement acceptable.

2. Procédé de préparation des composés de formule (I) selon la revendication 1 tels que X représente un radical (CH₃)₂N-CH₂-CH₂-O-C, leurs isomères, énantiomères, diastéréoisomères et épimères ainsi que leurs sels d'addition à un acide ou à une base pharmaceutiquement acceptable.

3. Procédé de préparation des composés de formule (I) selon la revendication 1 tels que X représente un atome d'azote, leurs énantiomères, diastéréoisomères et épimères ainsi que leurs sels d'addition à un acide ou à une base pharmaceutiquement acceptable.

4. Procédé de préparation des composés de formule (I) selon la revendication 1 tels que A représente un radical éthylène, leurs énantiomères, diastéréoisomères et épimères ainsi que leurs sels d'addition à un acide ou à une base pharmaceutiquement acceptable.

5. Procédé de préparation des composés de formule (I) selon la revendication 1 tels que Y et Z forment ensemble un atome d'oxygène, leurs énantiomères, diastéréoisomères et épimères ainsi que leurs sels d'addition à un acide ou à une base pharmaceutiquement acceptable.

6. Procédé de préparation du composé de formule (I) selon l'une quelconque des revendications 1, 2, 4 et 5 qui est le N,N'-di-[4-(2-diméthylaminoéthoxy)salicylidène]éthylène diamine, oxovanadium (IV), ainsi que ses sels d'addition à un acide pharmaceutiquement acceptable.

## Revendications (Revendications pour l'(les) Etat(s) contractant(s) suivant(s): ES)

1. Procédé de préparation des composés de formule (I) : 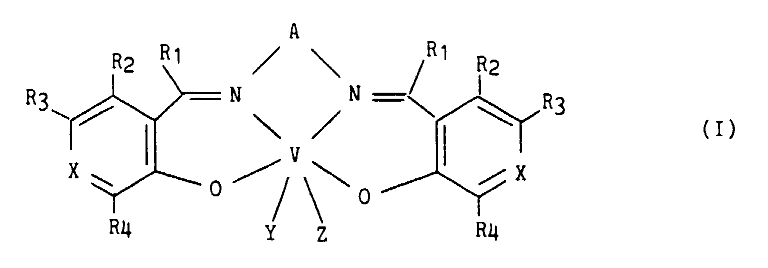 dans laquelle :
R₁ ou R₄, identiques ou différents, représentent un atome d'hydrogène ou un groupement alkyle (C₁-C₆) linéaire ou ramifié,
R₂ représente un atome d'hydrogène, un groupement alkyle (C₁-C₆) linéaire ou ramifié, un groupement hydroxyméthyl, un groupement -CH₂OPO(OH)₂ ou un groupement -CH₂OPO(ONa)₂,
R₃ représente un atome d'hydrogène, un groupement alkyle (C₁-C₆) linéaire ou ramifié, un groupement hydroxy ou l'un quelconque des groupements suivants :
-T-(CH₂)ₙ-CO₂H 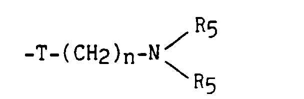 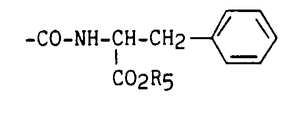
dans lesquels :
T représente un atome d'oxygène ou de soufre,
n représente un entier compris entre 1 et 4,
X représente un atome d'azote, un radical CH ou un radical CR'₃ (dans lequel R'₃ a la même signification que R₃ à l'exception du cas où R₃ représente un groupement hydroxy),
A représente un radical alkylène de formule -(CH₂)ₚ- dans lequel p représente un entier compris entre 2 et 4, éventuellement substitué par un ou plusieurs groupements alkyles (C₁-C₄) linéaires ou ramifiés, ou l'un quelconque des radicaux suivants : 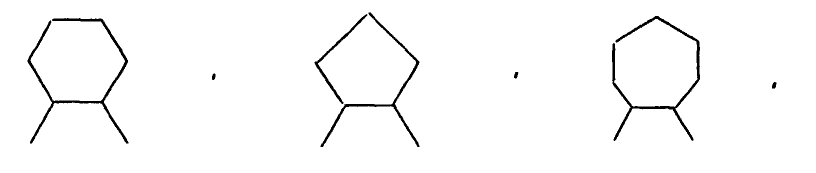
Y et Z forment ensemble un atome d'oxygène et dans ce cas le vanadium dans le complexe de formule (I) se trouve au degré d'oxydation IV
à la condition que :
lorsque :
X représente un radical CH ou C-alkyle,
alors R₃ est différent d'un atome d'hydrogène ou d'un groupement alkyle (C₁-C₆) linéaire ou ramifié,
leurs isomères, énantiomères, diastéréoisomères et épimères ainsi que leurs sels d'addition à un acide ou à une base pharmaceutiquement acceptable,
caractérisé en ce que l'on fait réagir deux équivalents d'un composé de formule (II) : 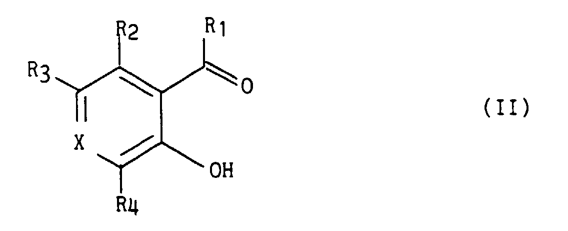 dans laquelle R₁, R₂, R₃ et R₄ ont la même signification que dans la formule (I),
avec un équivalent d'un composé de formule (III), sous atmosphère inerte : 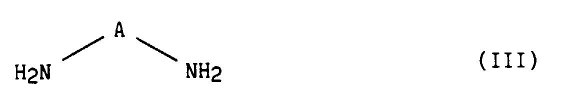 dans laquelle A a la même signification que dans la formule (I),
pour conduire au composé de formule (IV) dont on sépare éventuellement les isomères par une technique classique de séparation, que l'on purifie le cas échéant par une technique classique de purification : 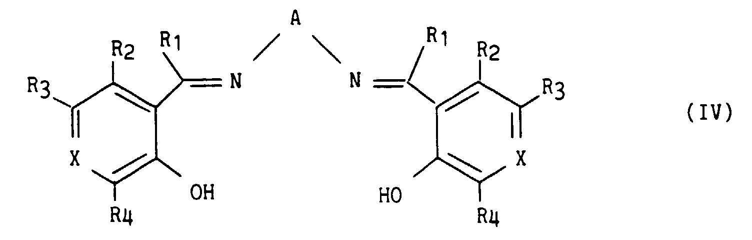 dans laquelle R₁, R₂, R₃, R₄, A et X ont la même signification que dans la formule (I),
que l'on traite :
- soit par une solution aqueuse pentahydratée de sulfate de vanadyle en milieu dichlorométhane, ou bien après traitement par de la soude par une solution aqueuse pentahydratée de sulfate de vanadyle en milieu tétrahydrofurane, ou bien par du sulfate de vanadyle en milieu diméthylformamide,
pour conduire au complexe de formule (I/a), cas particulier des composés de formule (I), dans lequel le vanadium se trouve au degré d'oxydation IV : 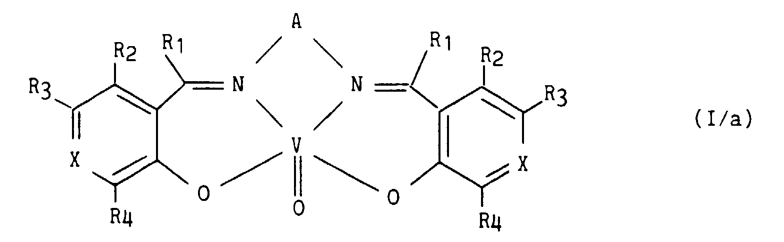
- soit par une solution alcoolique de sulfate de vanadyle en présence d'oxygène,
pour conduire au composé de formule (I/b), cas particulier des composés de formule (I), dans lequel le vanadium se trouve au degré d'oxydation V : 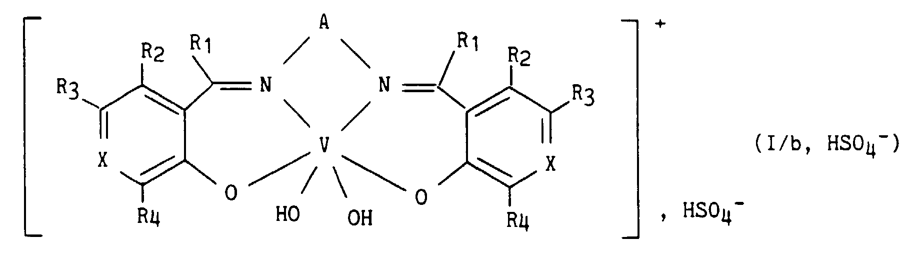 composés de formule (I/a) ou (I/b) que l'on purifie, le cas échéant, par une technique classique de purification et que l'on transforme, si on le désire, en leurs sels d'addition à un acide ou à une base pharmaceutiquement acceptable.

2. Procédé de préparation des composés de formule (I) selon la revendication 1 tels que X représente un radical (CH₃)₂N-CH₂-CH₂-O-C, leurs isomères, énantiomères, diastéréoisomères et épimères ainsi que leurs sels d'addition à un acide ou à une base pharmaceutiquement acceptable.

3. Procédé de préparation des composés de formule (I) selon la revendication 1 tels que X représente un atome d'azote, leurs énantiomères, diastéréoisomères et épimères ainsi que leurs sels d'addition à un acide ou à une base pharmaceutiquement acceptable.

4. Procédé de préparation des composés de formule (I) selon la revendication 1 tels que A représente un radical éthylène, leurs énantiomères, diastéréoisomères et épimères ainsi que leurs sels d'addition à un acide ou à une base pharmaceutiquement acceptable.

5. Procédé de préparation des composés de formule (I) selon la revendication 1 tels que Y et Z forment ensemble un atome d'oxygène, leurs énantiomères, diastéréoisomères et épimères ainsi que leurs sels d'addition à un acide ou à une base pharmaceutiquement acceptable.

6. Procédé de préparation du composé de formule (I) selon l'une quelconque des revendications 1, 2, 4 et 5 qui est le N,N'-di-[4-(2-diméthylaminoéthoxy)salicylidène]éthylène diamine, oxovanadium (IV), ainsi que ses sels d'addition à un acide pharmaceutiquement acceptable.

## Patentansprüche (Patentansprüche für folgende(n) Vertragsstaat(en): AT, BE, CH, DE, DK, FR, GB, IT, LI, LU, NL, PT, SE)

1. Verbindungen der Formel (I): 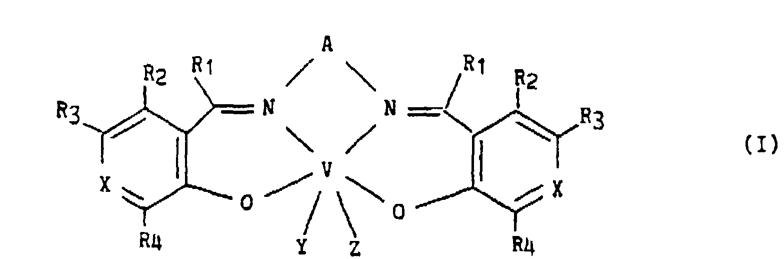 in der:
R₁ oder R₄, die gleichartig oder verschieden sind, ein Wasserstoffatom oder eine geradkettige oder verzweigte (C₁-C₆)-Alkylgruppe,
R₂ ein Wasserstoffatom, eine geradkettige oder verzweigte (C₁-C₆)-Alkylgruppe, eine Hydroxymethylgruppe, eine Gruppe -CH₂OPO(OH)₂ oder eine Gruppe -CH₂OPO(ONa)₂,
R₃ ein Wasserstoffatom, eine geradkettige oder verzweigte (C₁-C₆)Alkylgruppe, eine Hydroxylgruppe oder eine der folgenden Gruppen:
-T-(CH₂)ₙ-CO₂H 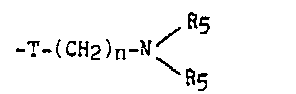 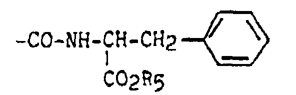
in denen:
T ein Sauerstoffatom oder ein Schwefelatom,
n eine ganze Zahl mit einem Wert zwischen 1 und 4 und
R₅ ein Wasserstoffatom oder eine geradkettige oder verzweigte (C₁-C₆)-Alkylgruppe darstellen.
X ein Stickstoffatom, eine Gruppe CH oder eine Gruppe CR'₃ (worin R'₃ die gleiche Bedeutung besitzt wie R₃ mit Ausnahme des Falls, da R₃ eine Hydroxylgruppe darstellt).
A eine Alkylengruppe der Formel -(CH₂)ₚ-, in der p eine ganze Zahl mit einem Wert zwischen 2 und 4 darstellt, die gegebenenfalls durch eine oder mehrere geradkettlge oder verzweigte (C₁-C₄)-Alkylgruppen substituiert ist, oder eine der folgenden Gruppen: 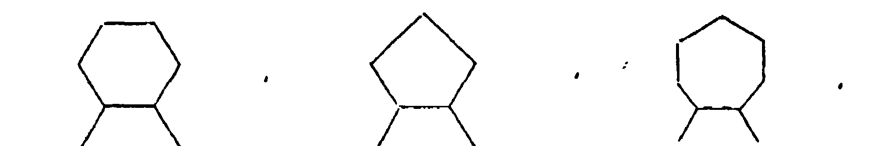
Y und Z gemeinsam ein Sauerstoffatom, wobei in diesem Fall das Vanadium in dem Komplex der Formel (I) sich in dem Oxidationszustand IV befindet.
mit der Maßgabe bedeuten, daß:
wenn:
X eine Gruppe CH oder C-Alkylgruppe darstellt, R₃ von einem Wasserstoffatom oder einer geradkettigen oder verzweigten (C₁-C₆)-Alkylgruppe verschieden ist.
deren Isomere, Enantiomere, Diastereoisomere und Epimere sowie deren Additionssalze mit einer pharmazeutisch annehmbaren Säure oder Base.

2. Verbindungen der Formel (I) nach Anspruch 1, worin X eine Gruppe (CH₃)₂N-CH₂-CH₂-O-C darstellt, deren Isomere, Enantiomere, Diastereoisomere und Epimere sowie deren Additionssalze mit einer pharmazeutisch annehmbaren Säure oder Base.

3. Verbindungen der Formel (I) nach Anspruch 1, worin X ein Stickstoffatom bedeutet, deren Enantiomere, Diastereoisomere und Epimere sowie deren Additionssalze mit einer pharmazeutisch annehmbaren Säure oder Base.

4. Verbindungen der Formel (I) nach Anspruch 1, worin A eine Ethylengruppe darstellt. deren Entiomere, Diastereoisomere und Epimere sowie deren Additionssalze mit einer pharmazeutisch annehbmaren Säure oder Base.

5. verbindungen der Formel (I) nach Anspruch 1, worin Y und Z gemeinsam ein Sauerstoffatom bilden. deren Enantiomere, Diastereoisomere und Epimere sowie deren Additionssalze mit einer pharmazeutisch annehmbaren Säure oder Base.

6. Verbindung der Formel (I) nach irgendeinem der Ansprüche 1, 2, 4 und 5, nämlich N,N'-Di-[4-(2-dimethylaminoethoxy)-salicyliden]-ethylendiamin-oxovanadium-(IV), sowie deren Additionssalze mit einer pharmazeutisch annehmbaren Säure.

7. Verfahren zur Herstellung der Verbindungen der Formel (I) nach Anspruch 1, **durch gekennzeichnet,** daß man zwei Äquivalente einer Verbindung der Formel (II): 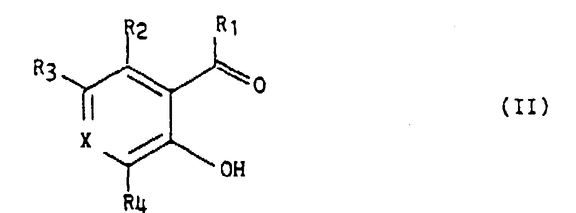 in der R₁, R₂, R₃ und R₄ die gleichen Bedeutungen wie bezüglich der Formel (I) besitzen,
mit einem Äquivalent einer Verbindung der Formel (III): 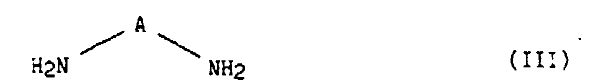 in der A die gleichen Bedeutungen besitzt wie bezüglich der Formel (I), unter inerter Atmosphäre umsetzt
zur Bildung der Verbindung der Formel (IV): 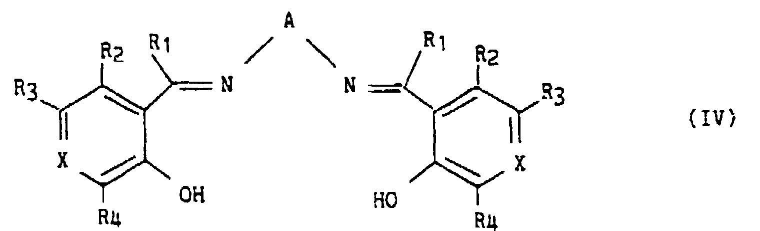 in der R₁, R₂, R₃, R₄, A und X die gleichen Bedeutungen wie bezüglich der Formel (I) besitzen. die man gegebenenfalls mit Hilfe einer klassischen Trennmethode in die Isomeren auftrennt und gegebenenfalls mit Hilfe einer klassischen Reinigungsmethode reinigt.
welche man:
- entweder mit einer wäßrigen Lösung von Vanadylsulfat-pentahydrat in Dichlormethanmedium oder nach der Behandlung mit Natriumhydroxid mit einer wäßrigen Lösung von Vanadylsulfat-pentahydrat in Tetrahydrofuranmedium oder mit Vanadylsulfat in Dimethylformamidmedium behandelt,
zur Bildung des Komplexes der Formel (I/a), einem Sonderfall der Verbindungen der Formel (I), in dem das Vanadium sich in dem Oxidationszustand (IV) befindet: 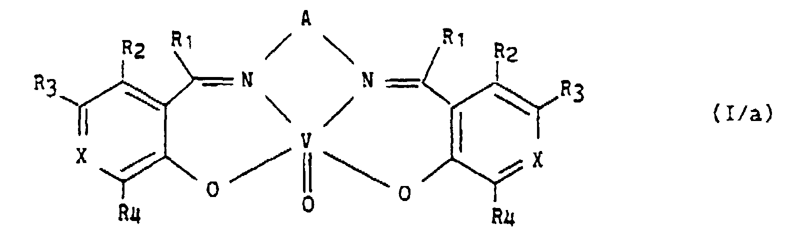
- oder mit einer alkoholischen Lösung von Vanadylsulfat in Gegenwart von Sauerstoff behandelt zur Bildung der Verbindung der Formel (I/b), einem Sonderfall der Verbindungen der Formel (I), in der das Vanadium sich in dem Oxidationszustand (V) befindet: 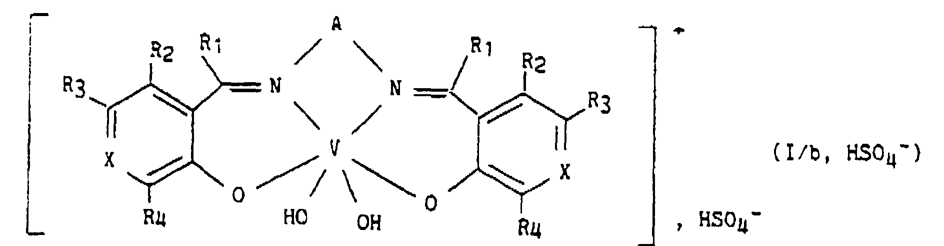
welche Verbindungen der Formeln (I/a) oder (I/b) man gegebenenfalls mit Hilfe einer klassischen Reinigungsmethode reinigt und gewünschtenfalls mit einer pharmazeutisch annehmbaren Säure oder Base in ihre Additionssalze umwandelt.

8. Pharmazeutische Zubereitungen enthaltend als Wirkstoff mindestens eine Verbindung nach einem der Ansprüche 1 bis 6 allein oder in Kombination mit einem oder mehreren inerten, nichttoxischen, pharmazeutisch annehmbaren Trägermaterialien oder Bindemitteln.

9. Pharmazeutische Zubereitungen nach Anspruch 8 enthaltend mindestens einen Wirkstoff nach einem der Ansprüche 1 bis 6 zur Behandlung des Diabetes, der Fettsucht und damit verknüpften pathologischen Zuständen.

10. Verwendung von N,N'-Di-(salicyliden)-ethylendiamin-oxovanadium-(IV), zur Herstellung einer pharmazeutischen Zubereitung nach Anspruch 8 zur Behandlung des Diabetes, der Fettsucht und damit verbundenen pathologischen Zuständen.

## Patentansprüche (Patentansprüche für folgende(n) Vertragsstaat(en): GR)

1. Verfahren zur Herstellung der Verbindungen der Formel (I): 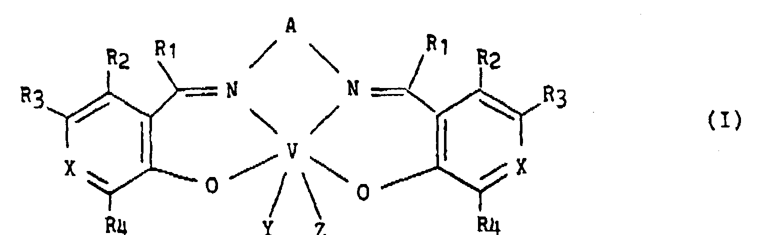 in der:
R₁ oder R₄, die gleichartig oder verschieden sind, ein Wasserstoffatom oder eine geradkettige oder verzweigte (C₁-C₆)-Alkylgruppe,
R₂ ein Wasserstoffatom, eine geradkettige oder verzweigte (C₁-C₆)-Alkylgruppe, eine Hydroxymethylgruppe, eine Gruppe -CH₂OPO(OH)₂ oder eine Gruppe -CH₂OPO(ONa)₂.
R₃ ein Wasserstoffatom, eine geradkettige oder verzweigte (C₁-C₆)-Alkylgruppe, eine Hydroxylgruppe oder eine der folgenden Gruppen:
T-(CH₂)ₙ-CO₂H 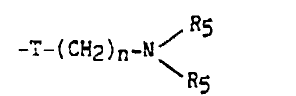 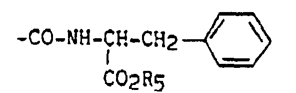
in denen:
T ein Sauerstoffatom oder ein Schwefelatom,
n eine ganze Zahl mit einem Wert zwischen 1 und 4 und
R₅ ein Wasserstoffatom oder eine geradkettige oder verzweigte (C₁-C₆)-Alkylgruppe darstellen,
X ein Stickstoffatom, eine Gruppe CH oder eine Gruppe CR'₃ (worin R'₃ die gleiche Bedeutung besitzt wie R₃ mit Ausnahme des Falls, da R₃ eine Hydroxylgruppe darstellt),
A eine Alkylengruppe der Formel -(CH₂)ₚ-, in der p eine ganze Zahl mit einem Wert zwischen 2 und 4 darstellt, die gegebenenfalls durch eine oder mehrere geradkettige oder verzweigte (C₁-C₄)-Alkylgruppen substituiert ist, oder eine der folgenden Gruppen: 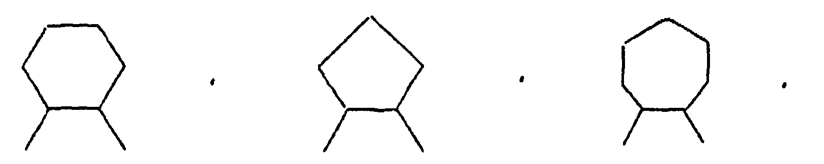
Y und Z gemeinsam ein Sauerstoffatom, wobei in diesem Fall das Vanadium in dem Komplex der Formel (I) sich in dem Oxidationszustand IV befindet.
mit der Maßgabe bedeuten, daß:
wenn:
X eine Gruppe CH oder C-Alkylgruppe darstellt, R₃ von einem Wasserstoffatom oder einer geradkettigen oder verzweigten (C₁-C₆)-Alkylgruppe verschieden ist,
von deren Isomeren, Enantiomeren, Diastereoisomeren und Epimeren sowie von deren Additionssalzen mit einer pharmazeutisch annehmbaren Säure oder Base. **dadurch gekennzeichnet**, daß man zwei Äquivalente einer verbindung der Formel (II): 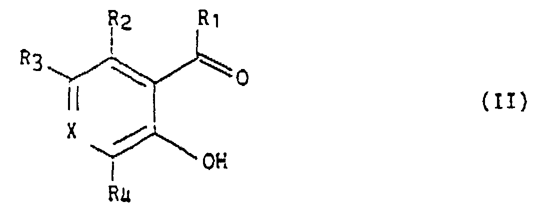 in der R₁, R₂, R₃ und R₄ die gleichen Bedeutungen wie bezüglich der Formel (I) besitzen,
mit einem Äquivalent einer Verbindung der Formel (III): 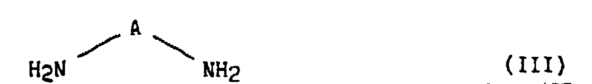 in der A die gleichen Bedeutungen besitzt wie bezüglich der Formel (I), unter inerter Atmosphäre umsetzt
zur Bildung der Verbindung der Formel (IV): 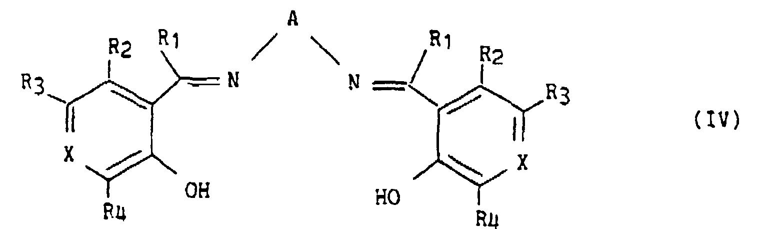 in der R₁, R₂, R₃, R₄, A und X die gleichen Bedeutungen wie bezüglich der Formel (I) besitzen,
die man gegebenenfalls mit Hilfe einer klassischen Trennmethode in die Isomeren auftrennt und gegebenenfalls mit Hilfe einer klassischen Reinigungsmethode reinigt,
welche man:
- entweder mit einer wäßrigen Lösung von Vanadylsulfat-pentahydrat in Dichlormethanmedium oder nach der Behandlung mit Natriumhydroxid mit einer wäßrigen Lösung von Vanadylsulfat- pentahydrat in Tetrahydrofuranmedium oder mit Vanadylsulfat in Dimethylformamidmedium behandelt,
zur Bildung des Komplexes der Formel (I/a), einem Sonderfall der Verbindungen der Formel (I), in dem das Vanadium sich im Oxidationszustand (IV) befindet: 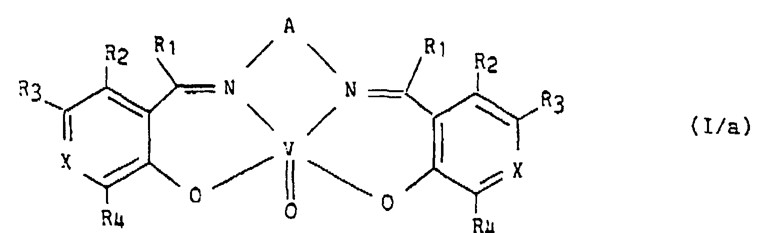
- oder mit einer alkoholischen Lösung von Vanadylsulfat in Gegenwart von Sauerstoff behandelt zur Bildung der Verbindung der Formel (I/b), einem Sonderfall der Verbindungen der Formel (I), in der das Vanadium sich in dem Oxidationszustand (V) befindet: 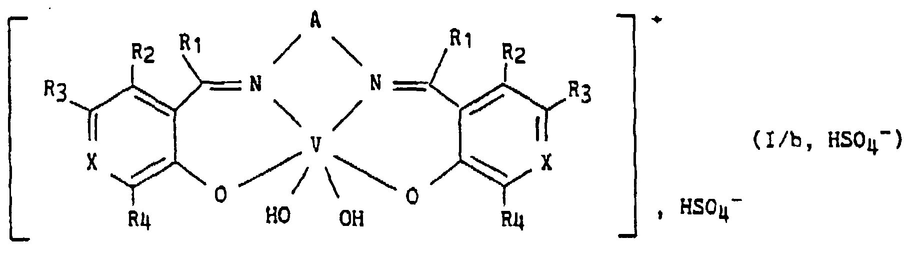
welche Verbindungen der Formeln (I/a) oder (I/b) man gegebenenfalls mit Hilfe einer klassischen Reinigungsmethode reinigt und gewünschtenfalls mit einer pharmazeutisch annehmbaren Säure oder Base in ihre Additionssalze umwandelt.

2. Verfahren zur Herstellung der Verbindungen der Formel (I) nach Anspruch 1, worin X eine Gruppe (CH₃)₂N-CH₂-CH₂-O-C darstellt, von deren Isomeren, Enantiomeren, Diastereoisomeren und Epimeren sowie von deren Additionssalzen mit einer pharmazeutisch annehmbaren Säure oder Base.

3. Verfahren zur Herstellung der Verbindungen der Formel (I) nach Anspruch 1, worin X ein stickstoffatom darstellt, von deren Enantiomeren, Diastereoisomeren und Epimeren sowie von deren Additionssalzen mit einer pharmazeutisch annehmbaren Säure oder Base.

4. Verfahren zur Herstellung der Verbindungen der Formel (I) nach Anspruch 1, worin A eine Ethylengruppe darstellt, von deren Entiomeren, Diastereoisomeren und Epimeren sowie von deren Additionssalzen mit einer pharmazeutisch annehbmaren Säure oder Base.

5. Verfahren zur Herstellung der Verbindungen der Formel (I) nach Anspruch 1, worin Y und Z gemeinsam ein Sauerstoffatom bilden, von deren Enantiomeren, Diastereoisomeren und Epimeren sowie von deren Additionssalzen mit einer pharmazeutisch annehmbaren Säure oder Base.

6. Verfahren zur Herstellung der Verbindung der Formel (I) nach irgendeinem der Ansprüche 1, 2, 4 und 5, nämlich von N,N'-Di-(4-(2-dimethylaminoethoxy)-salicyliden]-ethylendiamin-oxovanadium-(IV), sowie von deren Additionssalzen mit einer pharmazeutisch annehmbaren Säure.

## Patentansprüche (Patentansprüche für folgende(n) Vertragsstaat(en): ES)

1. Verfahren zur Herstellung der Verbindungen der Formel (I): 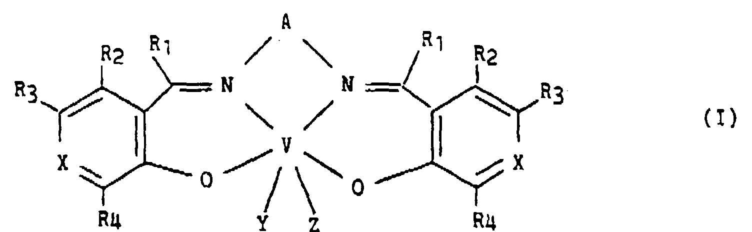 in der:
R₁ oder R₄, die gleichartig oder verschieden sind, ein Wasserstoffatom oder eine geradkettige oder verzweigte (C₁-C₆)-Alkylgruppe,
R₂ ein Wasserstoffatom, eine geradkettige oder verzweigte (C₁-C₆)-Alkylgruppe, eine Hydroxymethylgruppe, eine Gruppe -CH₂OPO(OH)₂ oder eine Gruppe -CH₂OPO(ONa)₂.
R₃ ein Wasserstoffatom, eine geradkettige oder verzweigte (C₁-C₆)-Alkylgruppe, eine Hydroxylgruppe oder eine der folgenden Gruppen:
-T-(CH₂)ₙ-CO₂H 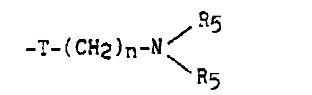 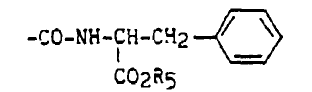
in denen:
T ein Sauerstoffatom oder ein Schwefelatom,
n eine ganze Zahl mit einem Wert zwischen 1 und 4 und
R₅ ein Wasserstoffatom oder eine geradkettige oder verzweigte (C₁-C₆)-Alkylgruppe darstellen,
X ein Stickstoffatom, eine Gruppe CH oder eine Gruppe CR'₃ (worin R'₃ die gleiche Bedeutung besitzt wie R₃ mit Ausnahme des Falls, da R₃ eine Hydroxylgruppe darstellt),
A eine Alkylengruppe der Formel -(CH₂)ₚ-, in der p eine ganze Zahl mit einem Wert zwischen 2 und 4 darstellt, die gegebenenfalls durch eine oder mehrere geradkettige oder verzweigte (C₁-C₄)-Alkylgruppen substituiert ist, oder eine der folgenden Gruppen: 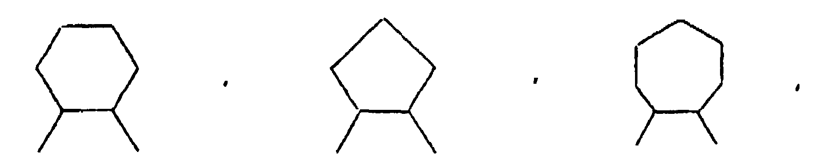
Y und Z gemeinsam ein Sauerstoffatom, wobei in diesem Fall das Vanadium in dem Komplex der Formel (I) sich in dem Oxidationszustand IV befindet.
mit der Maßgabe bedeuten, daß:
wenn:
X eine Gruppe CH oder C-Alkylgruppe darstellt, R₃ von einem Wasserstoffatom oder einer geradkettigen oder verzweigten (C₁-C₆)-Alkylgruppe verschieden ist.
von deren Isomeren, Enantiomeren, Diastereoisomeren und Epimeren sowie von d eren Additionssalzen mit einer pharmazeutisch annehmbaren Säure oder Base, **dadurch gekennzeichnet**, daß man zwei Äquivalente einer Verbindung der Formel (II): 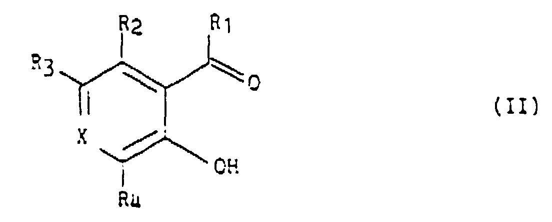 in der R₁, R₂, R₃ und R₄ die gleichen Bedeutungen wie bezüglich der Formel (I) besitzen,
mit einem Äquivalent einer Verbindung der Formel (III): 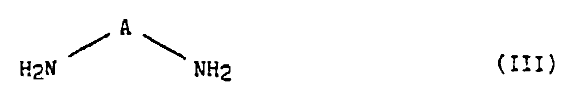 in der A die gleichen Bedeutungen besitzt wie bezüglich der Formel (I), unter inerter Atmosphäre umsetzt
zur Bildung der Verbindung der Formel (IV): 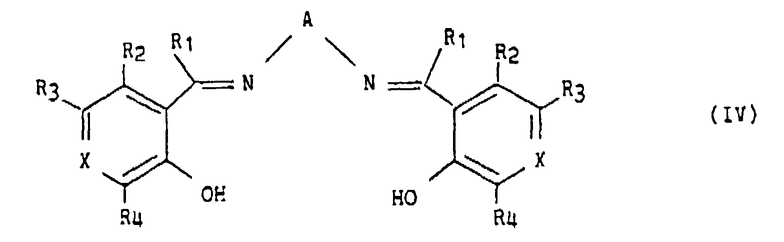 in der R₁, R₂, R₃, R₄, A und X die gleichen Bedeutungen wie bezüglich der Formel (I) besitzen,
die man gegebenenfalls mit Hilfe einer klassischen Trennmethode in die Isomeren auftrennt und gegebenenfalls mit Hilfe einer klassischen Reinigungsmethode reinigt,
welche man:
- entweder mit einer wäßrigen Lösung von Vanadylsulfat-pentahydrat in Dichlormethanmedium oder nach der Behandlung mit Natriumhydroxid mit einer wäßrigen Lösung von Vanadylsulfat-pentahydrat in Tetrahydrofuranmedium oder mit Vanadylsulfat in Dimethylformamidmedium behandelt.
zur Bildung des Komplexes der Formel (I/a), einem Sonderfall der Verbindungen der Formel (I), in dem das Vanadium sich im Oxidationszustand (IV) befindet: 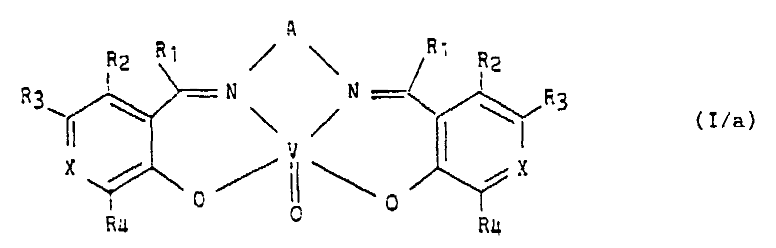
- oder mit einer alkoholischen Lösung von Vanadylsulfat in Gegenwart von Sauerstoff behandelt zur Bildung der Verbindung der Formel (I/b), einem Sonderfall der Verbindungen der Formel (I), in der das Vanadium sich in dem Oxidationszustand (V) befindet: 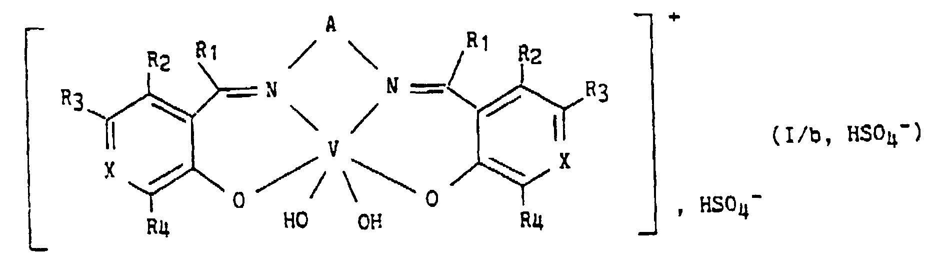
welche Verbindungen der Formeln (I/a) oder (I/b) man gegebenenfalls mit Hilfe einer klassischen Reinigungsmethode reinigt und gewünschtenfalls mit einer pharmazeutisch annehmbaren Säure oder Base in ihre Additionssalze umwandelt.

2. Verfahren zur Herstellung der Verbindungen der Formel (I) nach Anspruch 1, worin X eine Gruppe (CH₃)₂N-CH₂-CH₂-O-C darstellt, von deren Isomeren, Enantiomeren, Diastereoisomeren und Epimeren sowie von deren Additionssalzen mit einer pharmazeutisch annehmbaren Säure oder Base.

3. Verfahren zur Herstellung der Verbindungen der Formel (I) nach Anspruch 1, worin X ein Stickstoffatom darstellt, von deren Enantiomeren, Diastereoisomeren und Epimeren sowie von deren Additionssalzen mit einer pharmazeutisch annehmbaren Säure oder Base.

4. Verfahren zur Herstellung der Verbindungen der Formel (I) nach Anspruch 1, worin A eine Ethylengruppe darstellt, von deren Entiomeren, Diastereoisomeren und Epimeren sowie von deren Additionssalzen mit einer pharmazeutisch annehbmaren Säure oder Base.

5. Verfahren zur Herstellung der Verbindungen der Formel (I) nach Anspruch 1. worin Y und Z gemeinsam ein sauerstoffatom bilden, von deren Enantiomeren, Diastereoisomeren und Epimeren sowie von deren Additionssalzen mit einer pharmazeutisch annehmbaren Säure oder Base.

6. Verfahren zur Herstellung der Verbindung der Formel (I) nach irgendeinem der Ansprüche 1, 2, 4 und 5, nämlich von N,N'-Di-[4-[2-dimethylaminoethoxy)-salicyliden]-ethylendiamin-oxovanadium-(IV), sowie von deren Additionssalzen mit einer pharmazeutisch annehmbaren Säure.

## Claims (Claims for the following Contracting State(s): AT, BE, CH, DE, DK, FR, GB, IT, LI, LU, NL, PT, SE)

1. Compounds of formula (I): 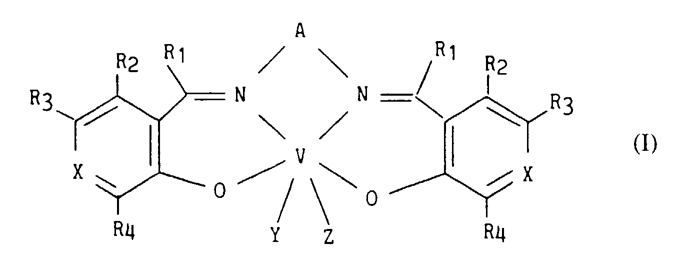 wherein:
R₁ and R₄, which may be the same or different, each represents a hydrogen atom or a linear or branched (C₁-C₆)alkyl group,
R₂ represents a hydrogen atom, a linear or branched (C₁-C₆)alkyl group, a hydroxymethyl group, a -CH₂OPO(OH)₂ group or a -CH₂OPO(ONa)₂ group,
R₃ represents a hydrogen atom, a linear or branched (C₁-C₆)alkyl group, a hydroxy group or any one of the following groups:
-T-(CH₂)ₙ-CO₂H 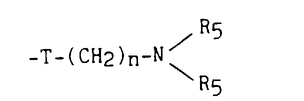 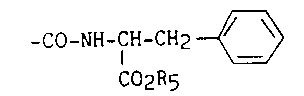
wherein:
T represents an oxygen or sulphur atom,
n represents an integer from 1 to 4,
R₅ represents a hydrogen atom or a linear or branched (C₁-C₆)alkyl group,
X represents a nitrogen atom, a CH radical or a CR'₃ radical (wherein R'₃ has the meaning given for R₃ with the exception of R₃ represents a hydroxy group),
A represents an alkylene radical of formula -(CH₂)ₚ- wherein p represents an integer from 2 to 4, optionally substituted by one or more linear or branched (C₁-C₄)alkyl groups, or represents any one of the following radicals: 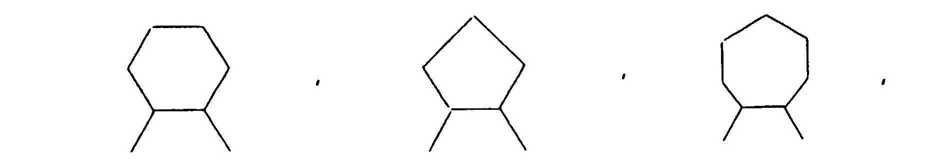
Y and Z together form an oxygen atom and in that case the vanadium in the complex of formula (I) has a degree of oxidation of IV
with the proviso that:
when:
X represents a CH or C-alkyl radical,
then R₃ is other than a hydrogen atom or a linear or branched (C₁-C₆)alkyl group,
their isomers, enantiomers, diastereoisomers and epimers and also the addition salts thereof with a pharmaceutically acceptable acid or base.

2. Compounds of formula (I) according to claim 1 wherein X represents a (CH₃)₂N-CH₂-CH₂-O-C radical, their isomers, enantiomers, diastereoisomers and epimers and also the addition salts thereof with a pharmaceutically acceptable acid or base.

3. Compounds of formula (I) according to claim 1 wherein X represents a nitrogen atom, their enantiomers, diastereoisomers and epimers and also the addition salts thereof with a pharmaceutically acceptable acid or base.

4. Compounds of formula (I) according to claim 1 wherein A represents an ethylene radical, their enantiomers, diastereoisomers and epimers and also the addition salts thereof with a pharmaceutically acceptable acid or base.

5. Compounds of formula (I) according to claim 1 wherein Y and Z together form an oxygen atom, their enantiomers, diastereoisomers and epimers and also the addition salts thereof with a pharmaceutically acceptable acid or base.

6. Compound of formula (I) according to any one of claims 1, 2, 4 and 5 that is N,N'-di[4-(2-dimethylaminoethoxy)salicylidene]ethylenediamine-oxovanadium(IV), and also the addition salts thereof with a pharmaceutically acceptable acid.

7. Process for the preparation of the compounds of formula (I) according to claim 1, characterised in that two equivalents of a compound of formula (II): 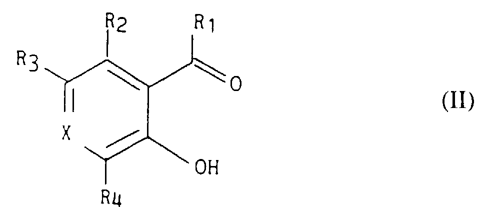 wherein R₁, R₂, R₃ and R₄ are as defined for formula (I),
are reacted, under an inert atmosphere, with one equivalent of a compound of formula (III): 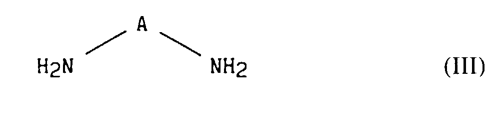 wherein A is as defined for formula (I),
to yield a compound of formula (IV) which is optionally separated into its isomers using a conventional separating method and which is purified, where appropriate, using a conventional purification method: 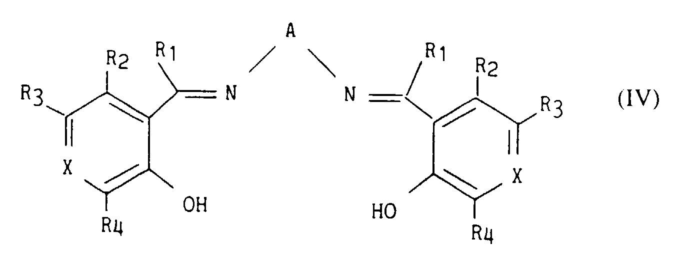 wherein R₁, R₂, R₃, R₄, A and X are as defined for formula (I),
which is treated:
- either with an aqueous solution of vanadyl sulphate pentahydrate in a dichloromethane medium or, after treatment with sodium hydroxide, with an aqueous solution of vanadyl sulphate pentahydrate in a tetrahydrofuran medium, or with vanadyl sulphate in a dimethylformamide medium,
to yield a complex of formula (I/a), a particular case of the compounds of formula (I), wherein the vanadium has a degree of oxidation of IV: 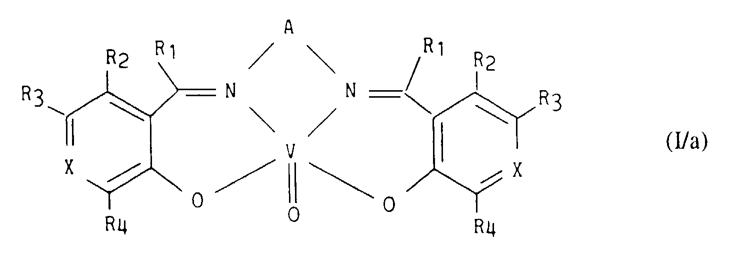
- or with an alcoholic solution of vanadyl sulphate in the presence of oxygen,
to yield a compound of formula (I/b), a particular case of the compounds of formula (I), wherein the vanadium has a degree of oxidation of V: 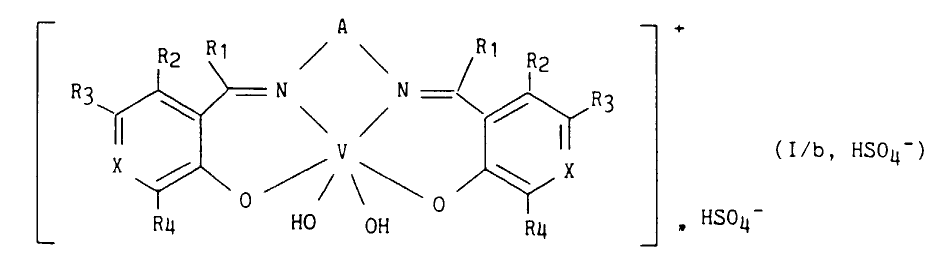 which compounds of formula (I/a) or (Vb) are purified, where appropriate, using a conventional purification method and are converted, if desired, into their addition salts with a pharmaceutically acceptable acid or base.

8. Pharmaceutical compositions comprising as active ingredient at least one compound according to any one of claims 1 to 6, alone or in combination with one or more pharmaceutically acceptable, non-toxic, inert excipient(s) or carrier(s).

9. Pharmaceutical compositions according to claim 8 comprising at least one active ingredient according to any one of claims 1 to 6 for use in the treatment of diabetes, obesity and associated pathologies.

10. Use of N,N'-di(salicylidene)ethylenediamine-oxovanadium(IV) for the preparation of a pharmaceutical composition according to claim 8 that can be used in the treatment of diabetes, obesity and associated pathologies.

## Claims (Claims for the following Contracting State(s): GR)

1. Process for the preparation of compounds of formula (I): 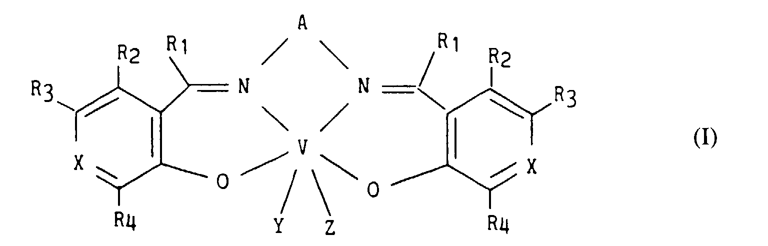 wherein:
R₁ and R₄, which may be the same or different, each represents a hydrogen atom or a linear or branched (C₁-C₆)alkyl group,
R₂ represents a hydrogen atom, a linear or branched (C₁-C₆)alkyl group, a hydroxymethyl group, a -CH₂OPO(OH)₂ group or a -CH₂OPO(ONa)₂ group,
R3 represents a hydrogen atom, a linear or branched (C₁-C₆)alkyl group, a hydroxy group or any one of the following groups:
-T-(CH₂)ₙ-CO₂H 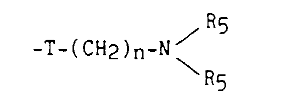 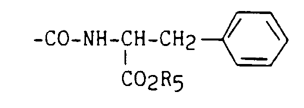
wherein:
T represents an oxygen or sulphur atom,
n represents an integer from 1 to 4,
R₅ represents a hydrogen atom or a linear or branched (C₁-C₆)alkyl group,
X represents a nitrogen atom, a CH radical or a CR'₃ radical (wherein R'₃ has the meaning given for R₃ with the exception of R₃ represents a hydroxy group),
A represents an alkylene radical of formula -(CH₂)ₚ- wherein p represents an integer from 2 to 4, optionally substituted by one or more linear or branched (C₁-C₄)alkyl groups, or represents any one of the following radicals: 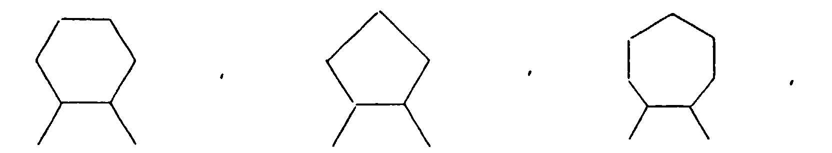
Y and Z together form an oxygen atom and in that case the vanadium in the complex of formula (I) has a degree of oxidation of IV
with the proviso that:
when:
X represents a CH or C-alkyl radical,
then R₃ is other than a hydrogen atom or a linear or branched (C₁-C₆)alkyl group,
their isomers, enantiomers, diastereoisomers and epimers and also the addition salts thereof with a pharmaceutically acceptable acid or base,
characterised in that two equivalents of a compound of formula (II): 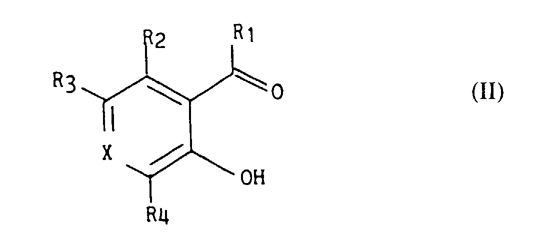 wherein R₁, R₂, R₃ and R₄ are as defined for formula (I),
are reacted, under an inert atmosphere, with one equivalent of a compound of formula (III): 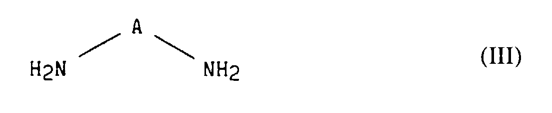 wherein A is as defined for formula (I),
to yield a compound of formula (IV) which is optionally separated into its isomers using a conventional separating method and which is purified, where appropriate, using a conventional purification method: 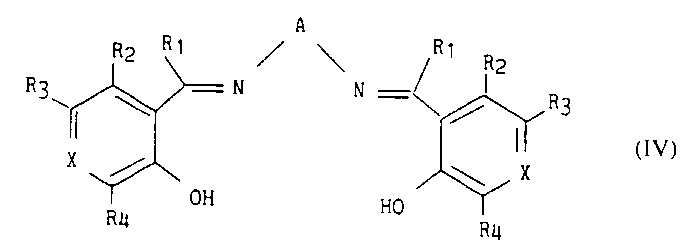 wherein R₁, R₂, R₃, R₄, A and X are as defined for formula (I),
which is treated:
- either with an aqueous solution of vanadyl sulphate pentahydrate in a dichloromethane medium or, after treatment with sodium hydroxide, with an aqueous solution of vanadyl sulphate pentahydrate in a tetrahydrofuran medium, or with vanadyl sulphate in a dimethylformamide medium,
to yield a complex of formula (I/a), a particular case of the compounds of formula (I), wherein the vanadium has a degree of oxidation of IV: 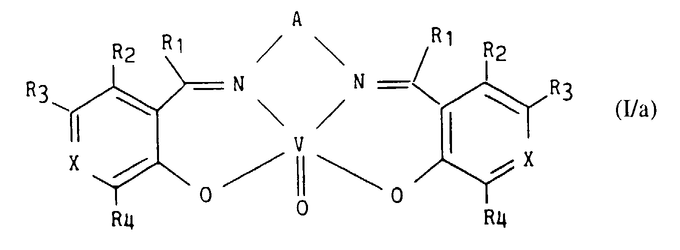
- or with an alcoholic solution of vanadyl sulphate in the presence of oxygen, to yield a compound of formula (I/b), a particular case of the compounds of formula (I), wherein the vanadium has a degree of oxidation of V: 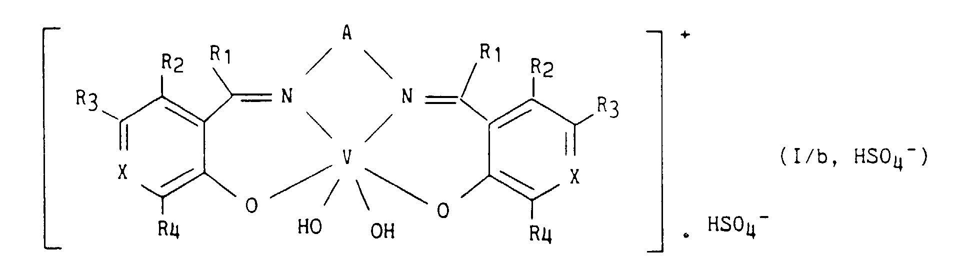
which compounds of formula (I/a) or (I/b) are purified, where appropriate, using a conventional purification method and are converted, if desired, into their addition salts with a pharmaceutically acceptable acid or base.

2. Process for the preparation of compounds of formula (I) according to claim 1 wherein X represents a (CH₃)₂N-CH₂-CH₂-O-C radical, their isomers, enantiomers, diastereoisomers and epimers and also the addition salts thereof with a pharmaceutically acceptable acid or base.

3. Process for the preparation of compounds of formula (I) according to claim 1 wherein X represents a nitrogen atom, their enantiomers, diastereoisomers and epimers and also the addition salts thereof with a pharmaceutically acceptable acid or base.

4. Process for the preparation of compounds of formula (I) according to claim 1 wherein A represents an ethylene radical, their enantiomers, diastereoisomers and epimers and also the addition salts thereof with a pharmaceutically acceptable acid or base.

5. Process for the preparation of compounds of formula (I) according to claim 1 wherein Y and Z together form an oxygen atom, their enantiomers, diastereoisomers and epimers and also the addition salts thereof with a pharmaceutically acceptable acid or base.

6. Process for the preparation of a compound of formula (I) according to any one of claims 1, 2, 4 and 5 that is N,N'-di[4-(2-dimethylaminoethoxy)salicylidene]-ethylenediamine-oxovanadium(IV), and also the addition salts thereof with a pharmaceutically acceptable acid.

## Claims (Claims for the following Contracting State(s): ES)

1. Process for the preparation of compounds of formula (I): 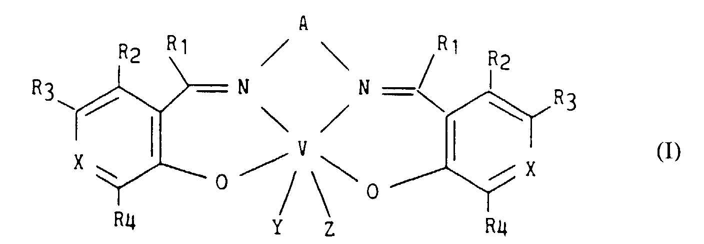 wherein:
R₁ and R₄, which may be the same or different, each represents a hydrogen atom or a linear or branched (C₁-C₆)alkyl group,
R₂ represents a hydrogen atom, a linear or branched (C₁-C₆)alkyl group, a hydroxymethyl group, a -CH₂OPO(OH)₂ group or a -CH₂OPO(ONa)₂ group,
R₃ represents a hydrogen atom, a linear or branched (C₁-C₆)alkyl group, a hydroxy group or any one of the following groups:
-T-(CH₂)ₙ-CO₂H 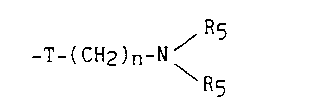 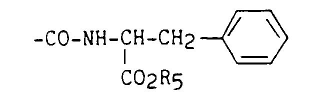
wherein:
T represents an oxygen or sulphur atom,
n represents an integer from 1 to 4,
R₅ represents a hydrogen atom or a linear or branched (C₁-C₆)alkyl group,
X represents a nitrogen atom, a CH radical or a CR'₃ radical (wherein R'₃ has the meaning given for R₃ with the exception of R₃ represents a hydroxy group),
A represents an alkylene radical of formula -(CH₂)ₚ- wherein p represents an integer from 2 to 4, optionally substituted by one or more linear or branched (C₁-C₄)alkyl groups, or represents any one of the following radicals: 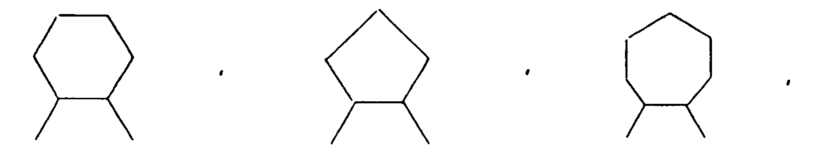
Y and Z together form an oxygen atom and in that case the vanadium in the complex of formula (I) has a degree of oxidation of IV
with the proviso that:
when:
X represents a CH or C-alkyl radical,
then R₃ is other than a hydrogen atom or a linear or branched (C₁-C₆)alkyl group,
their isomers, enantiomers, diastereoisomers and epimers and also the addition salts thereof with a pharmaceutically acceptable acid or base,
characterised in that two equivalents of a compound of formula (II): 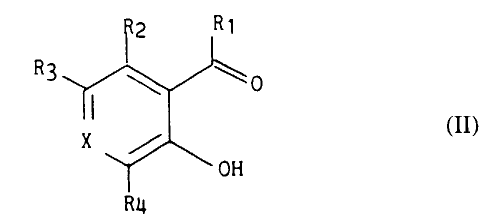 wherein R₁, R₂, R₃ and R₄ are as defined for formula (I),
are reacted, under an inert atmosphere, with one equivalent of a compound of formula (III): 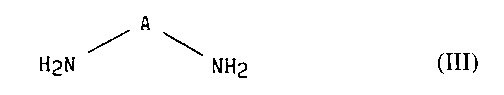 wherein A is as defined for formula (I),
to yield a compound of formula (IV) which is optionally separated into its isomers using a conventional separating method and which is purified, where appropriate, using a conventional purification method: 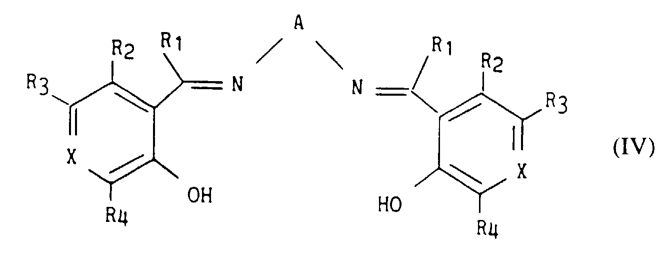 wherein R₁, R₂, R₃, R₄, A and X are as defined for formula (I),
which is treated:
- either with an aqueous solution of vanadyl sulphate pentahydrate in a dichloromethane medium or, after treatment with sodium hydroxide, with an aqueous solution of vanadyl sulphate pentahydrate in a tetrahydrofuran medium, or with vanadyl sulphate in a dimethylformamide medium,
to yield a complex of formula (I/a), a particular case of the compounds of formula (I), wherein the vanadium has a degree of oxidation of IV: 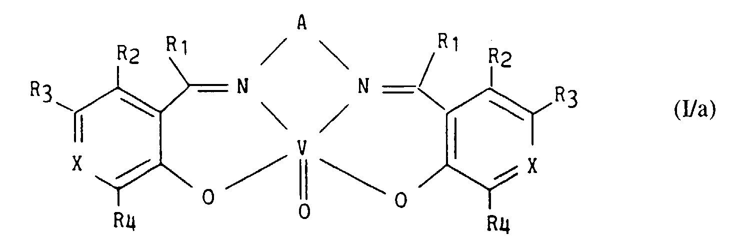
- or with an alcoholic solution of vanadyl sulphate in the presence of oxygen,
to yield a compound of formula (I/b), a particular case of the compounds of formula (I), wherein the vanadium has a degree of oxidation of V: 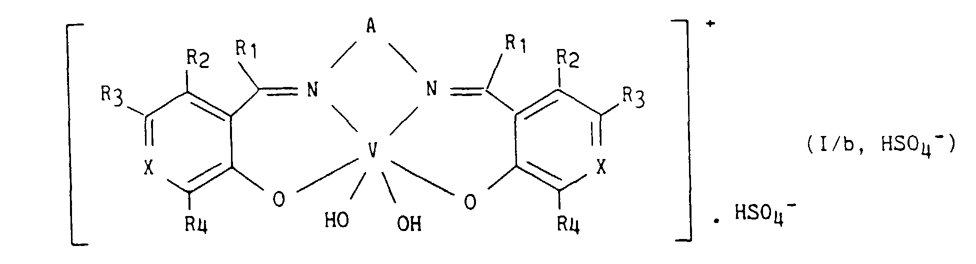 which compounds of formula (I/a) or (I/b) are purified, where appropriate, using a conventional purification method and are converted, if desired, into their addition salts with a pharmaceutically acceptable acid or base.

2. Process for the preparation of compounds of formula (I) according to claim 1 wherein X represents a (CH₃)₂N-CH₂-CH₂-O-C radical, their isomers, enantiomers, diastereoisomers and epimers and also the addition salts thereof with a pharmaceutically acceptable acid or base.

3. Process for the preparation of compounds of formula (I) according to claim 1 wherein X represents a nitrogen atom, their enantiomers, diastereoisomers and epimers and also the addition salts thereof with a pharmaceutically acceptable acid or base.

4. Process for the preparation of compounds of formula (I) according to claim 1 wherein A represents an ethylene radical, their enantiomers, diastereoisomers and epimers and also the addition salts thereof with a pharmaceutically acceptable acid or base.

5. Process for the preparation of compounds of formula (I) according to claim 1 wherein Y and Z together form an oxygen atom, their enantiomers, diastereoisomers and epimers and also the addition salts thereof with a pharmaceutically acceptable acid or base.

6. Process for the preparation of a compound of formula (I) according to any one of claims 1, 2, 4 and 5 that is N,N'-di[4-(2-dimethylaminoethoxy)salicylidene]-ethylenediamine-oxovanadium(IV), and also the addition salts thereof with a pharmaceutically acceptable acid.
